# FASCICULE DE BREVET EUROPEEN

(11) **EP 1 756 579 B1**
(45) Date de publication et mention de la délivrance du brevet: **03.03.2010**
(21) Numéro de dépôt: 05778653.5
(22) Date de dépôt: 15.06.2005
(51) Int. Cl.: G01N 33/58, G01N 33/543, G01N 33/566, C07K 1/13

(54) **COLLECTIONS DE COMPOSES TRACABLES ET LEURS UTILISATIONS**
SAMMLUNGEN AUS VERFOLGBAREN VERBINDUNGEN UND VERWENDUNG DAFÜR
COLLECTIONS OF TRACEABLE COMPOUNDS AND USES THEREOF

(30) Priorité: 15.06.2004 FR 0406462
(43) Date de publication de la demande: 28.02.2007
(73) Titulaire: CENTRE NATIONAL DE LA RECHERCHE SCIENTIFIQUE (CNRS), 75794 Paris Cedex 16 (FR); Université Louis Pasteur de Strasbourg, F-67000 Strasbourg (FR)
(72) Inventeur: HIBERT, Marcel, François, Louis, F-67114 Eschau (FR); FRANCHET, Christel, Anne, F-67120 Ernolsheim-Bruche (FR); GALZI, Jean-Luc, F-67500 Weitbruch (FR); PATTUS, Franc, Emile, Jean, F-67000 Strasbourg (FR); GUILLIER, Fabrice, Yves, F-67400 Illkirch Graffenstaden (FR)
(74) Mandataire: Faivre Petit, Frédérique
(86) Numéro de dépôt international: PCT/FR2005/001502
(87) Numéro de publication internationale: WO 2006/003330

(56) Documents cités:
- WO-A-00/23463
- WO-A-97/07402
- FR-A- 2 764 387
- US-A- 4 900 658
- US-A- 5 854 216
- US-A- 5 916 877
- US-A1- 2002 034 766
- CIVELLI ET AL: "Orphan GPCRs and their ligands" PHARMACOLOGY AND THERAPEUTICS, vol. 110, 2006, pages 525-532,
- KOTARSKY ET AL: "Reverse pharmacology and the de-orphanization of 7TM receptors" DRUG DISCOVERY TODAY, no. 2, 2004, pages 99-104,
- POOGA ET AL: "cell penetration by transportan" FASEB, vol. 12, 1998, pages 67-77,

## Description

La présente invention a pour objet des collections de composés traçables, ainsi que leurs utilisations, notamment dans le cadre de la détermination de ligands d'un récepteur dont on ne connaît pas de ligand ou dont on ne connaît pas de ligand utilisable pour les études de liaison d'affinité spécifique.

Le gène codant pour une protéine fluorescente issue de la méduse *Aequorea victoria*, la protéine fluorescente verte (green fluorescent protein ou GFP) (Prasher et al. 1992, Gene 111:229-233), a été récemment déchiffré. La GFP est une protéine monomérique. Elle acquiert ses propriétés de fluorescence par un mécanisme autocatalytique de formation du fluorophore.

De nombreux médicaments et substances naturelles exercent leur action en interagissant avec des protéines régulatrices appelées récepteurs, impliquées dans de nombreuses fonctions physiologiques des organismes, et les altérations de leurs fonctions sont la cause de nombreuses pathologies. L'accessibilité des récepteurs aux agents pharmacologiques endogènes ou exogènes, naturels ou synthétiques, depuis l'extérieur de la cellule conduit à les considérer comme des cibles dé choix pour la recherche de molécules biologiquement actives, en particulier de molécules présentant des pouvoirs thérapeutiques potentiels.

Le décryptage du génome humain donne accès à la séquence de centaines de protéines nouvelles dont on ne connaît ni le ligand endogène ni la fonction biologique. Ces protéines, dites "orphelines", constituent les sites potentiels d'action de médicaments.

Les méthodes existantes à ce jour ne permettent de détecter que des molécules agonistes qui activent une fonction associée à la protéine. Ce type d'essai fonctionnel conduit à de nombreux faux positifs coûteux en temps et en argent. Par ailleurs, les molécules antagonistes qui inhiberaient la fonction associée à la protéine ne peuvent pas être détectées directement par ces méthodes. Or, les antagonistes présentent la plupart du temps le meilleur potentiel thérapeutique.

FR2764387 décrit l'utilisation d'une protéine fluorescente pour la détection d'une interaction entre une protéine cible, comme par exemple un récepteur couplé au protéines G, et son ligand par FRET (fluorescence energy transfer). La visualisation de cette interaction est exemplifiée avec la bodipy-neurokine et le récepteur NK2R-GFP.

La présente invention a pour but de proposer un procédé de détermination d'un ligand quelconque, qu'il soit endogène ou exogène, permettant de s'affranchir de la recherche systématique du ligand endogène ou naturel.

L'un des buts de l'invention est de fournir un procédé de criblage du premier ligand d'un récepteur orphelin sans connaître son ligand endogène.

La présente invention concerne l'utilisation d'une collection de composés traçables de type cationique, pour la détermination de ligands d'un récepteur dont on ne connaît pas de ligand ou dont on ne connaît pas de ligand utilisable pour les études de liaison d'affinité spécifique,
lesdits composés traçables de type cationique étant **caractérisés en ce qu**'ils comprennent au moins un résidu d'acide aminé à caractère basique conférant le type cationique audit composé et un groupement traceur, notamment un fluorophore, un colorant ou un "quencher".

L'expression "composés traçables de type cationique" désigne des molécules contenant un groupe chargé positivement dans les conditions physiologiques.

L'expression "récepteur dont on ne connaît pas de ligand" désigne toute macromolécule biologique ("récepteur") pour laquelle on ne connaît pas de molécules endogène ou exogène venant s'y lier de manière réversible.

L'expression "liaison d'affinité spécifique" désigne la mesure de la force d'interaction entre deux molécules, à savoir un récepteur et son ligand.

L'expression "récepteur dont on ne connaît pas de ligand utilisable pour les études de liaison d'affinité spécifique" désigne toute macromolécule biologique (récepteur) pour laquelle il n'existe pas encore de molécule pouvant s'y lier (ligand) de manière suffisamment spécifique et détectable.

L'expression "résidu d'acide aminé à caractère basique" désigne un résidu d'acide aminé (alpha-, beta-, gamma-, delta-aminé, ou autre) contenant une entité protonable dans les conditions physiologiques.

L'expression "groupe traceur" désigne une entité chimique fluorescente ou une entité chimique possédant des propriétés de colorant ou d'absorption de certaines longueurs d'onde.

Une utilisation avantageuse selon l'invention est **caractérisée en ce que** les composés traçables de type cationique comprennent au moins un groupement "aa-cation-espaceur-fluorophore" ou "cation-aa-espaceur-fluorophore", "aa" correspondant à un résidu d'acide aminé, "cation" correspondant à un résidu d'acide aminé à caractère basique, "espaceur" correspondant à un groupe espaceur et "fluorophore" correspondant à un groupe fluorophore, ledit groupement étant **caractérisé en ce que** l'entité "aa" et l'entité "cation" sont reliées entre elles par une liaison peptidique ou pseudo-peptidique.

L'expression "liaison peptidique" désigne une liaison amide -CO-NH- entre deux acides aminés naturels.

L'expression "liaison pseudo-peptidique" désigne une liaison -CO-NH- entre deux acides aminés non naturels.

L'expression "groupe espaceur" désigne une entité séparant le groupe traceur du reste de la molécule.

La présente invention concerne l'utilisation d'une collection de composés traçables de type cationique répondant à la formule (I-1) suivante : 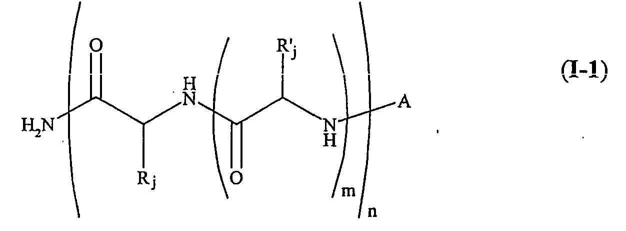 dans laquelle :
- m est égal à 0 ou 1,
- n représente un nombre entier variant de 1 à 10, et notamment de 1 à 6, et étant de préférence égal à 2 ou à 3,
- j représente un nombre entier variant de 1 à n,
- Rⱼ et R'ⱼ représentent une chaîne latérale d'acide aminé, l'un au moins des Rⱼ et R'ⱼ représentant une chaîne latérale d'acide aminé à caractère basique, et, lorsque m = 1, au moins deux des groupes Rⱼ représentent une chaîne latérale d'acide aminé à caractère basique et tous les R'ⱼ sont différents d'une chaîne latérale d'acide aminé à caractère basique,
- A représente un groupement traceur qui est une entité chimique fluorescente ou une entité chimique possédant des propriétés de colorant ou d'absorption de certaines longueurs d'onde, ou un groupe de la forme D-G, D représentant un groupe espaceur qui est une entité séparant le groupe traceur du reste de la molécule et G représentant un groupe traceur tel que défini précédemment,
pour la détermination *in vitro* de ligands d'un récepteur dont on ne connaît pas de ligand ou dont on ne connaît pas de ligand utilisable pour les études de liaison d'affinité spécifique.

Une utilisation avantageuse selon la présente invention est **caractérisée en ce que** les composés traçables de type cationique répondent à la formule (I) suivante : 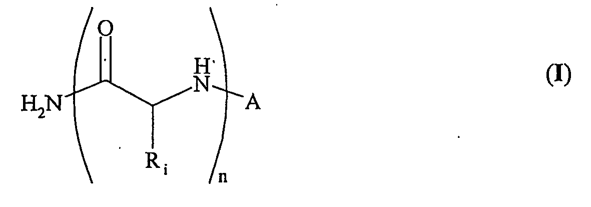 dans laquelle :
- n représente un nombre entier variant de 1 à 10, et notamment de 1 à 6, et étant de préférence égal à 2 ou à 3,
- i représente un nombre entier variant de 1 à n,
- Rᵢ représente une chaîne latérale d'acide aminé, l'un au moins des Rᵢ représentant une chaîne latérale d'acide aminé à caractère basique, les Rᵢ pouvant tous être identiques ou différents,
- A représente un groupement traceur tel que défini dans la revendication 1 ou un groupe de la forme D-G, D représentant un groupe espaceur et G représentant un groupe traceur tel que défini précédemment.

La présente invention concerne également les sels des composés traçables de type cationique de formule (I) susmentionnée.

La présente invention concerne également l'utilisation telle que définie ci-dessus, **caractérisée en ce que** les composés traçables de type cationique répondent à la formule suivante : 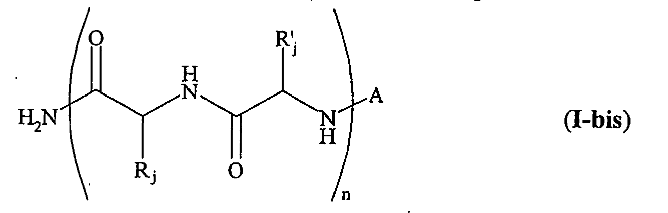 dans laquelle :
- n représente un nombre entier variant de 2 à 10, et notamment de 2 à 6, et étant de préférence égal à 2 ou à 3,
- j répond à la définition donnée précédemment pour i dans les composés de formule (I),
- Rⱼ et R'ⱼ représentent une chaîne latérale d'acide aminé, **caractérisés en ce qu**'au moins deux des groupes Rⱼ représentent une chaîne latérale d'acide aminé à caractère basique et en ce que tous les R'ⱼ soient différents d'une chaîne latérale d'acide aminé à caractère basique, les Rⱼ pouvant tous être identiques ou différents et les R'ⱼ pouvant tous être identiques ou différents, et
- A est tel que défini ci-dessus, et représente de préférence un fluorophore.

La présente invention concerne également un composé de formule (I-1) suivante : 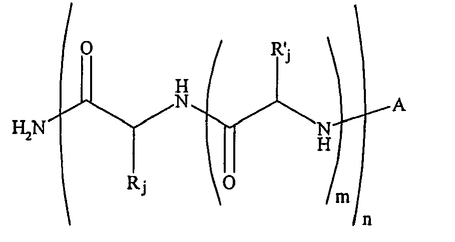 dans laquelle :
- m est égal à 0 ou 1,
- n représente un nombre entier variant de 1 à 10, et notamment de 1 à 6, et étant de préférence égal à 2 ou à 3,
- i représente un nombre entier variant de 1 à n,
- Rⱼ et R'ⱼ représentent une chaîne latérale d'acide aminé, l'un au moins des Rⱼ et R'ⱼ représentant une chaîne latérale d'acide aminé à caractère basique, et, lorsque m = 1, au moins deux des groupes Rⱼ représentent une chaîne latérale d'acide aminé à caractère basique et tous les R'ⱼ sont différents d'une chaîne latérale d'acide aminé à caractère basique,
- A représente un groupement traceur, notamment un fluorophore, un colorant ou un "quencher", ou un groupe de la forme D-G, D représentant un groupe espaceur et G représentant un groupe traceur tel que défini précédemment.

Un composé particulièrement avantageux selon l'utilisation et le procédé de la présente invention est un composé de formule (I-bis), **caractérisé en ce que** seulement deux des groupes Rⱼ représentent une chaîne latérale d'acide aminé à caractère basique.

De tels composés correspondent à des composés traçables de type biscationique qui ont une forte probabilité de se lier à certaines classes de récepteurs ciblés.

Un composé avantageux selon la présente invention est un composé de formule (I-bis) telle que définie ci-dessus, **caractérisé en ce que** :
- Rⱼ représente une chaîne latérale d'acide aminé à caractère basique, et de préférence la chaîne latérale de la lysine, de l'ornithine ou de l'arginine,
- R'ⱼ représente une chaîne latérale d'acide aminé, ledit acide aminé étant de préférence choisi dans le groupe comprenant : l'alanine, la glycine, l'acide 6-aminocaproïque, la leucine, la glutamine, l'acide glutamique, la méthionine, la proline, l'acide isonipécotique, l'acide carboxylique de tétraisoquinoline, l'acide 3-aminobenzoïque, l'acide 4-aminométhylbenzoïque, le tryptophane, l'histidine, la phénylalanine, la cyrosine, la 2-naphtylalanine, la phénylalanine de benzoyle,

Selon un mode de réalisation préféré, le composé répond à la formule (I), **caractérisé en ce que** n est égal à 2, et répondant à la formule (II) suivante : 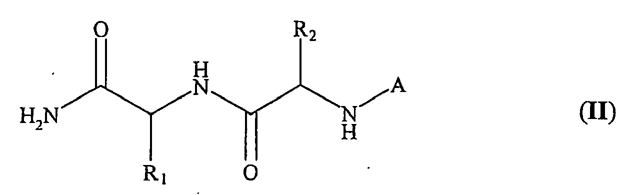 dans laquelle :
- A est tel que défini ci-dessus, et représente de préférence un fluorophore,
- R₁ représente une chaîne latérale d'acide aminé à caractère basique, et de préférence la chaîne latérale de la lysine, de l'ornithine ou de l'arginine, et
- R₂ représente une chaîne latérale d'acide aminé, ledit acide aminé étant de préférence choisi dans le groupe comprenant : l'alanine, la glycine, l'acide 6-aminocaproïque, la leucine, la glutamine, l'acide glutamique, la méthionine, la proline, l'acide isonipécotique, l'acide carboxylique de tétraisoquinoline, l'acide 3-aminobenzoïque, l'acide 4-aminométhylbenzoïque, le tryptophane, l'histidine, la phénylalanine, la tyrosine, la 2-naphtylalanine, la phénylalanine de benzoyle.

Les composés susmentionnés de formule (II) sont des composés monocationiques.

Selon un mode de réalisation préféré, le composé de formule (I), est **caractérisé en ce que** n est égal à 3, et répondant à la formule (III) suivante : 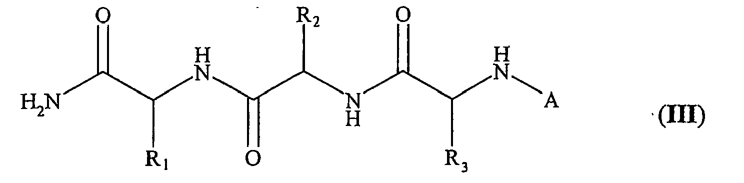 dans laquelle :
- A est tel que défini ci-dessus, et représente de préférence un fluorophore,
- R₁ et R₃ représentent une chaîne latérale d'acide aminé à caractère basique, et de préférence la chaîne latérale de la lysine, de l'ornithine ou de l'arginine, et
- R₂ représente une chaîne latérale d'acide aminé, ledit acide aminé étant de préférence choisi dans le groupe comprenant : l'alanine, la glycine, l'acide 6-aminocaproïque, la leucine, la glutamine, l'acide glutamique, la méthionine, la proline, l'acide isonipécotique, l'acide carboxylique de tétraisoquinoline, l'acide 3-aminobenzoïque, l'acide 4-aminométhylbenzoïque, le tryptophane, l'histidine, la phénylalanine, la tyrosine, la 2-naphtylalanine, la phénylalanine de benzoyle.

Les composés susmentionnés de formule (III) sont des composés biscationiques. Un composé avantageux est un composé tel que défini ci-dessus, **caractérisé en ce que** le groupe espaceur D est choisi parmi les groupes de formule suivante :

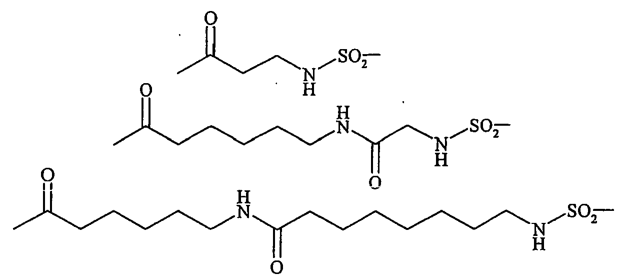

La présente invention concerne également l'utilisation d'un composé tel que défini ci-dessus, de formule (I), (I-bis), (II) ou (III), **caractérisé en ce que** A représente un groupe fluorophore dont les longueurs d'onde d'absorption et d'émission sont compatibles avec le processus de résonance par transfert d'énergie de fluorescence avec les divers mutants de la protéine fluorescente verte, ledit fluorophore étant notamment choisi parmi le groupe comprenant le Bodipy et la lissamine.

Un composé préféré est un composé tel que défini ci-dessus, de formule (I), (I-bis), (II) ou (III), **caractérisé en ce que** A représente l'un des groupes suivants :

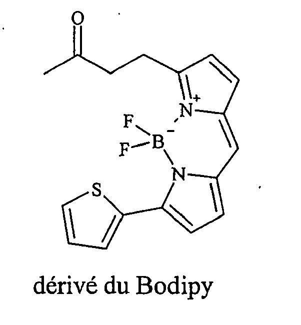

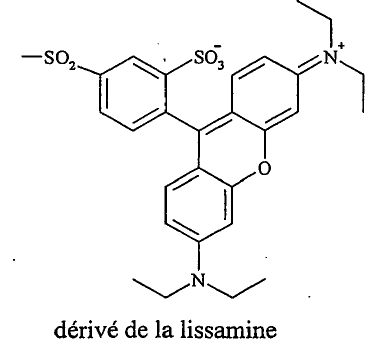

L'expression "collection" désigne un ensemble de molécules préparées selon le même protocole.

La présente invention concerne également l'utilisation d'une collection telle que définie ci-dessus, pour la détermination de ligands d'un récepteur dont on ne connaît pas de ligand ou dont on ne connaît pas de ligand utilisable pour les études de liaison d'affinité spécifique.

La présente invention concerne également un procédé de criblage de ligands de récepteurs dont on ne connaît pas de ligand ou dont on ne connaît pas de ligand utilisable, ledit procédé comprenant les étapes suivantes :
- la mise en présence d'une collection de composés traçables telle que défini ci-dessus, avec des cellules transfectées par une construction contenant la fusion de la séquence codant pour une protéine fluorescente, avec la séquence nucléotidique codant pour un récepteur dont on ne connaît pas de ligand ou dont on ne connaît pas de ligand utilisable, et le mélange desdites cellules et de ladite collection,
- la détection de la fluorescence dudit mélange, par excitation de ladite protéine fluorescente et mesure de la fluorescence d'émission de ladite protéine fluorescente, et la détermination du pourcentage d'extinction de fluorescence en comparant la fluorescence d'émission de ladite protéine fluorescente dans le mélange à la fluorescence moyenne de ladite protéine fluorescente en l'absence de ligand,
   la fluorescence moyenne de ladite protéine fluorescente en absence de ligand étant mesurée par des essais témoins correspondant à la mesure de la fluorescence de la protéine fluorescente en l'absence de la collection de composés, et
- la détermination des composés qui donnent un pourcentage d'extinction de la fluorescence de la protéine fluorescente d'au moins 5% et leur identification en tant que ligand.

La présente invention concerne plus particulièrement un procédé de criblage de ligands de récepteurs couplés aux protéines G (RCPG) tels que GPR50, GPR37, GPR19, GPR15, GPR31, GPR81, GPR3 et EDG7, le récepteur APJ, FPRL1, les récepteurs des chimiokines CXCR1, CXCR2, CXCR3, CXCR4, les récepteurs des peptides Glucagon-like (GLP-1R et GLP-2R), les récepteurs CRF1 et CRF2, les récepteurs métabotropiques au glutamate (mGluR) et les récepteurs au GABA.

La présente invention concerne également un procédé de criblage de ligands de RCPG dont on ne connaît pas de ligands tels que les récepteurs orphelins GPR50, GPR37, GPR19, GPR31, GPR81, GPR3 et EDG7.

La présente invention concerne aussi un procédé de criblage de ligands de RCPG dont on ne connaît pas de ligands utilisables selon le procédé de l'invention tels que le récepteur APJ, FPRL1, les récepteurs des chimiokines, CXCR1, CXCR2, CXCR3, CXCR4, les récepteurs des peptides Glucagon-like (GLP-1R et GLP2-R), CRF1, CRF2, les récepteurs métabotropiques au glutamate (mGluR) et les récepteurs au GABA.

### DESCRIPTION DES FIGURES

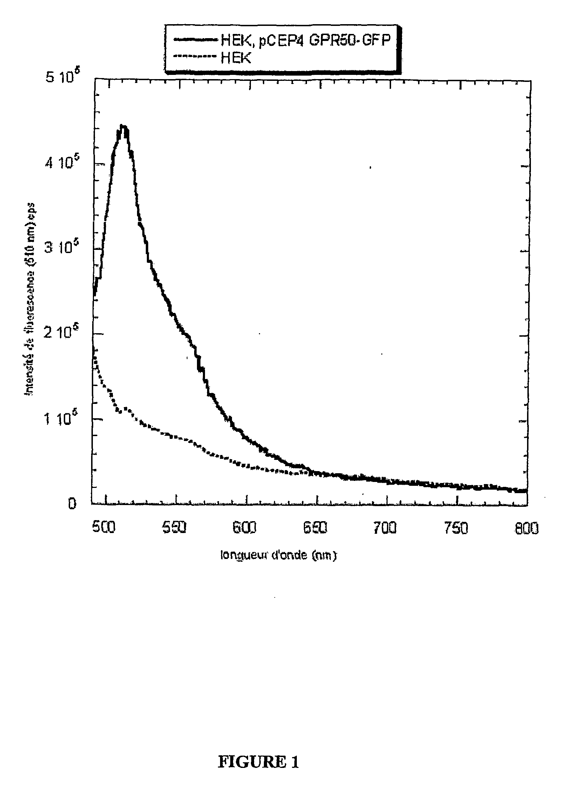

La Figure 1 représente un spectre d'émission de cellules HEK 293 transfectées par la construction pCEP4-SP-EGFP-GPR50. L'axe des ordonnées correspond à la longueur d'onde (en nm) et l'axe des abscisses à l'intensité de fluorescence (en cps). La courbe en trait plein correspond aux cellules HEK exprimant pCEP4-SP-EGFP-GPR50 et la courbe en trait pointillé aux cellules HEK non transfectées.

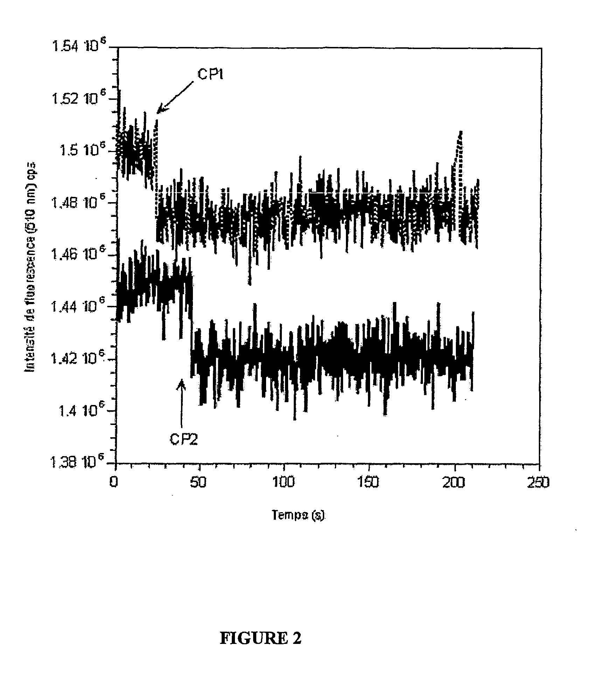

La Figure 2 représente la mesure en temps réel du transfert d'énergie entre des ligands peptidiques fluorescents ci-dessous (CP1 et CP2 ; 1 µM) et le récepteur GPR50-EGFP. En abscisse est représenté le temps en seconde et en ordonnée l'intensité de fluorescence à 510 nm (coups par seconde), l'excitation ayant lieu à 470 nm (un faible transfert d'énergie de l'ordre de 5 % peut être mesuré).

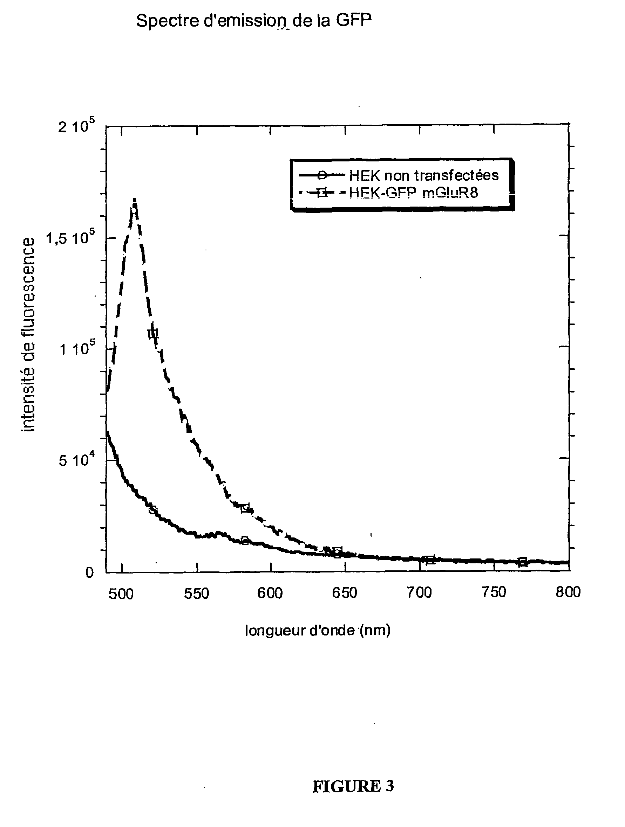

La Figure 3 représente un spectre d'émission de la GFP (excitation 475 nm). L'intensité de fluorescence de cellules HEK exprimant le récepteur GFP-m GluR8 est comparé par rapport à des cellules non transfectées. La figure représente un spectre d'émission de cellules HEK 293 transfectées par la construction pcDNA6-SP-GFP-mGluR8. L'axe des ordonnées correspond à la longueur d'onde (en nm) et l'axe des abscisses à l'intensité de fluorescence (en cps). La courbe en pointillés correspond aux cellules HEK exprimant pcDNA6-SP-GFP-mGluR8 et la courbe en trait plein aux cellules HEK non transfectées.

### PARTIE EXPÉRIMENTALE

### Préparation de tripeptides fluorescents - Schéma général

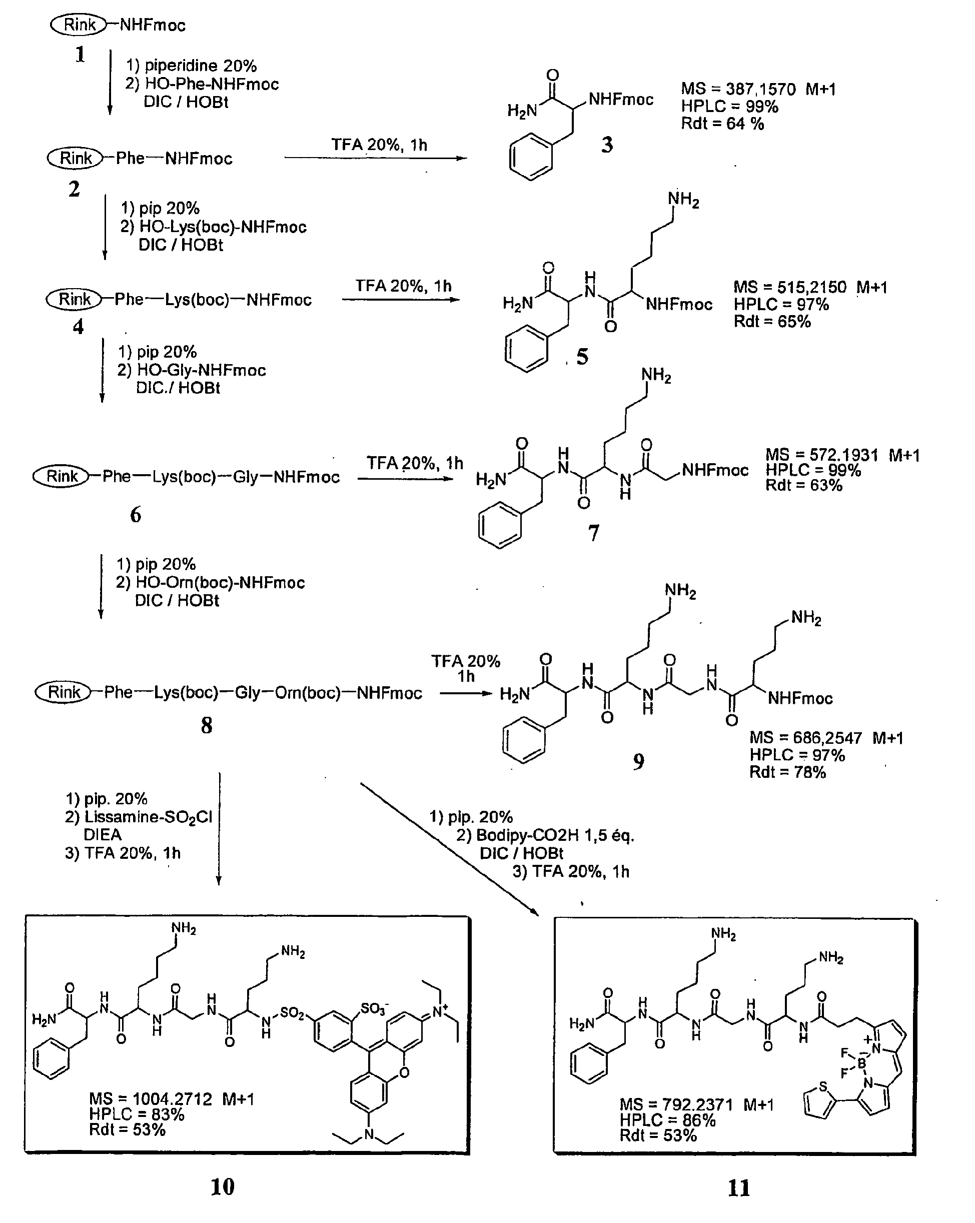

### PROTOCOLE EXPÉRIMENTAL

### I - MODES OPÉRATOIRES GÉNÉRAUX

### 1) Mode opératoire général (A) de greffage d'un acide aminé sur une résine Rink

### Synthèse de la résine Rink-Phe-NHFmoc (CHPO 72A ou 116A ; composé 1)

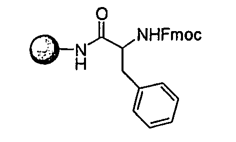

Dans un ballon, la résine Rink-NHFmoc (5 g ; 3 mmol ; 0,6 mmol/g) est traitée avec 50 mL d'une solution de 20% de pipéridine dans le DMF et agitée pendant 20 min. Le mélange est filtré sur fritté et la résine est lavée avec deux séquences de 50 mL de DMF, DCM, MeOH et une séquence de 50 mL de DMF, DCM. (résine CHPO 24A)

Une solution d'acide aminé préactivé est préparée en mélangeant dans l'ordre N-α-Fmoc-L-phénylalanine (569 mg ; 1,455 mmol) et HOBt (222 mg ; 1,455 mmol) dans 3 mL de DMF/DCM (1:1), pendant 15 minutes à température ambiante. Le DIC (230 µL ; 1,455 mmol) est ajouté à la solution d'acide carboxylique et de HOBt et le mélange est agité pendant 15 minutes à température ambiante. Pendant ce temps, la résine déprotégée Rink-NH₂ CHPO 24A (700 mg ; 0,485 mmol ; 0,693 mmol/g) est expansée avec 5 mL de DMF/DCM (1:4) dans un ballon, pendant 30 minutes. La solution d'acide carboxylique préactivée (1,455 mmol ; 3 éq.) est ajoutée à la résine. L'ensemble est agité pendant 14 heures à température ambiante. La résine est filtrée sur un fritté, lavée avec deux séquences de 10 mL de DMF, DCM, MeOH et une séquence de 10 mL de DMF, DCM, puis séchée sous pression réduite pendant 4 heures.

### 2) Mode opératoire général (B) de greffage du Bodipy sur la résine Rink

### Synthèse de la résine Rink-Phe-Bodipy (CHPO 78A)

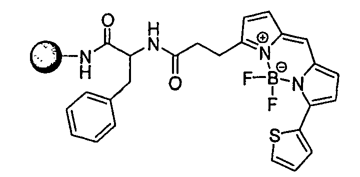

Dans une seringue Supelco munie d'un fritté, la résine Rink-Phe-NHFmoc (677 mg ; 0,374 mmol ; 0,552 mmol/g) est traitée avec 7 mL d'une solution de 20% de pipéridine dans le DMF et agitée par rotation pendant 20 minutes. Le mélange est filtré et la résine est lavée avec deux séquences de 7 mL de DMF, DCM, MeOH et une séquence de 7 mL de DMF, DCM (résine CHPO 77A).

Une solution d'acide carboxylique préactivée est préparée en mélangeant dans l'ordre l'acide 4,4-difluoro-5-(2-thiényl)-4-bora-3a,4a-diaza-*s*-indacène-3-propionique CHPO65C (52 mg; 0,151 mmol) et HOBt (23 mg; 0,151 mmol) dans 1 mL de DMF/DCM (1:1), pendant 15 minutes à température ambiante. Le DIC (24 µL ; 0,151 mmol) est ajouté à la solution d'acide carboxylique et de HOBt et le mélange est agité pendant 15 minutes à température ambiante. Pendant ce temps, la résine déprotégée Rink-Phe-NH₂ CHPO 77A (200 mg ; 0,126 mmol ; 0,629 mmol/g) est expansée 30 minutes avec 2 mL de DMF/DCM (1:4) dans une seringue Supelco munie d'un fritté. La solution d'acide carboxylique préactivée (0,151 mmol ; 1,2 éq.) est ajoutée à la résine. L'ensemble est agité par rotation pendant 14 heures à température ambiante. La résine est filtrée sur un fritté, lavée avec deux séquences de 3 mL de DMF, DCM, MeOH et une séquence de 3 mL de DMF, DCM, puis séchée sous pression réduite pendant 4 heures.

### 3) Mode opératoire général (C) du clivage de molécules fluorescentes

### Synthèse de H₂N-CO-Phe-Bodipy (CHPO 79A)

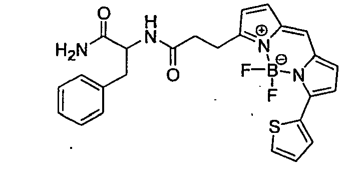

Dans une seringue Supelco, la résine Rink-Phe-Bodipy CHPO 78A (240 mg ; 0,126 mmol ; 0,521 mmol/g) est traitée avec 2 mL de TFA/DCM/H₂O (20:75:5) pendant 3 heures à température ambiante. La résine est filtrée, lavée avec du DCM (3 × 3 mL). Le filtrat est collecté et amené à sec sous pression réduite pour fournir 50 mg de solide violet intense.

Rdt = 80%, HPLC 81 % (dionex, tr = 29,59 min ; λ. = 288 nm ; gradient t = 0 min : 2% de CH₃CN dans H₂O (0,1% TFA) t = 30 min : 100% CH₃CN t = 40 min : 100% ; 0,5 mL/min). MS (ESI-TOF) *m*/*z* (M+H) calculé pour C₂₅H₂₃BF₂N₄O₂S + H, 493 ; trouvé, 493. RMN ¹H (CDCl₃) δ 8,12-8,13 (m, 1H) ; 7,50-7,52 (m, 1H) ; 7,11-7,27 (m, 8H) ; 7,05-7,06 (m, 1H) ; 6,93-6,94 (m, 1H) ; 6,80-6,81 (m, 1H) ; 4,75-4,76 (m, 1H) ; 3,28-3,29 (m, 2H) ; 2,94-2,95 (m, 2H) ; 2,67-2,68 (m, 2H).

### 4) Mode opératoire général (D) de greffage de la Lissamine sur la résine Rink

### Synthèse de la résine Rink-Phe-Lissamine (CHPO 112A)

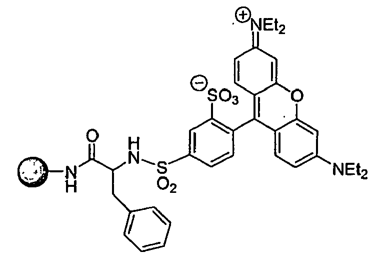

Dans une seringue Supelco munie d'un fritté, la résiné Rink-Phe-NHFmoc (630 mg ; 0,350 mmol ; 0,556 mmol/g) est traitée avec 7 mL d'une solution de 20% de pipéridine dans le DMF et agitée par rotation pendant 20 minutes. Le mélange est filtré et la résine est lavée avec deux séquences de 7 mL de DMF, DCM, MeOH et une séquence de 7 mL de DMF, DCM. (résine CHPO 99B).

Dans une seringue Supelco munie d'un fritté, la résine Rink-Phe-NH₂ (50 mg ; 0,032 mmol ; 0,635 mmol/g) est expansée avec 1 mL de DCM. La DIEA (11 µL ; 0,064 mmol ; 2 éq.) est ajoutée, puis la Lissamine rhodamine B sulfonyl chloride (37 mg ; 0,064 mmol ; 2 éq.) dissoute dans 2 mL de DCM. Le mélange réactionnel est agité 5 heures à température ambiante. La résine est filtrée, lavée avec deux séquences de 3 mL de DMF, DCM, MeOH et une séquence de 3 mL de DMF, DCM, puis séchée sous pression réduite pendant 4 heures.

### 5) Synthèse de H₂N-CO-Phe-Lissamine (CHPO 112B)

### (selon le mode opératoire général (C))

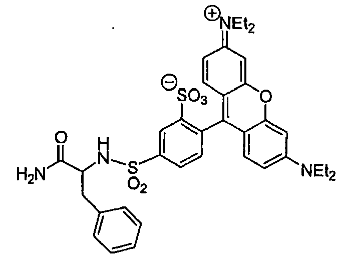

Dans une seringue Supelco, la résine Rink-Phe-lissamine CHPO 112A (20 mg ; 0,042 mmol ; 0,473 mmol/g) est traitée avec 2 mL de TFA/DCM/H₂O (20:75:5) pendant 2 h 30 à température ambiante. La résine est filtrée, lavée avec du DCM (3 × 3 mL). Le filtrat est collecté et amené à sec sous pression réduite pour fournir un solide violet intense.

HPLC 70% (dionex, tr = 27,77 min; λ = 288 nm ; gradient t : 0 min : 2% de CH₃CN dans H₂O (0,1% TFA) t = 30 min : 100% CH₃CN t = 40 min : 100% ; 0,5 mL/min). MS (ESI-TOF) *m*/*z* (M+H) calculé pour C₃₆H₄₀N₄O₇S₂ + H, 705 ; trouvé, 705.

### 6) Synthèse de la résine Rink-Arg(pmc)-NHFmoc (CHPO 74A)

### (selon le mode opératoire général (A))

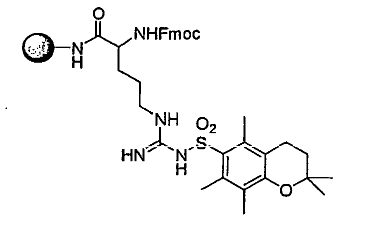

Dans un ballon, la résine Rink-NHFmoc (5 g ; 3 mmol ; 0,6 mmol/g) est traitée avec 50 mL d'une solution de 20% de pipéridine dans le DMF et agitée pendant 20 minutes. Le mélange est filtré sur fritté et la résine est lavée avec deux séquences de 50 mL de DMF, DCM, MeOH et une séquence de 50 mL de DMF, DCM (résine CHPO 24A).

Une solution d'acide aminé préactivé est préparée en mélangeant dans l'ordre N-α-Fmoc-N-ω-(2,2,5,7,8-pentaméthylchromane-6-sulfonyl)-L-Arginine (1,102 g ; 1,663 mmol) et HOBt (254 mg ; 1,663 mmol) dans 10 mL de DMF/DCM (1:1), pendant 15 minutes à température ambiante. Le DIC (262 µL ; 1,663 mmol) est ajouté à la solution d'acide carboxylique et de HOBt et le mélange est agité pendant 15 minutes à température ambiante. Pendant ce temps, la résine déprotégée Rink-NH₂ CHPO 24A (1,2 g ; 0,832 mmol ; 0,693 mmol/g) est expansée avec 10 mL de DMF/DCM (1:4) dans un ballon, pendant 30 minutes. La solution d'acide carboxylique préactivée (1,455 mmol ; 3 éq.) est ajoutée à la résine. L'ensemble est agité pendant 14 heures à température ambiante. La résine est filtrée sur un fritté, lavée avec deux séquences de 20 mL de DMF, DCM, MeOH et une séquence de 20 mL de DMF, DCM, puis séchée sous pression réduite pendant 4 heures.

### 7) Synthèse de la résine Rink-Arg(pmc)-Lissamine (CHPO 113A)

### (selon le mode opératoire général (D))

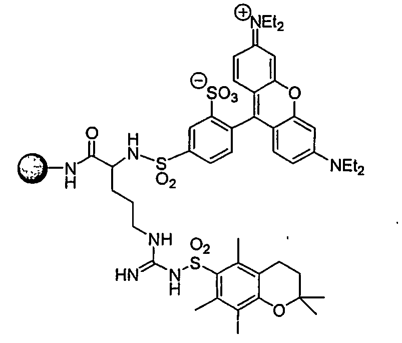

Dans une seringue Supelco munie d'un fritté, la résine Rink-Arg(pmc)-NHFmoc CHPO 74A (852 mg ; 0,408 mmol ; 0.479 mmol/g) est traitée avec 7 mL d'une solution de 20% de pipéridine dans le DMF et agitée par rotation pendant 20 minutes. Le mélange est filtré et la résine est lavée avec deux séquences de 7 mL de DMF, DCM, MeOH et une séquence de 7 mL de DMF, DCM (résine CHPO 80A).

Dans une seringue Supelco munie d'un fritté, la résine Rink-Arg(pmc)-NH₂ CHPO80A (50 mg ; 0,027 mmol ; 0,536 mmol/g) est expansée avec 1 mL de DCM. La DIEA (9 µL ; 0,054 mmol ; 2 éq.) est ajoutée, puis la Lissamine rhodamine B sulfonyl chloride (31 mg ; 0,054 mmol ; 2 éq.) dissoute dans 2 mL de DCM. Le mélange réactionnel est agité 5 heures à température ambiante. La résine est filtrée, lavée avec deux séquences de 3 mL de DMF, DCM, MeOH et une séquence de 3 mL de DMF, DCM, puis séchée sous pression réduite pendant 4 heures.

### 8) Synthèse de H₂N-CO-Arg-Lissamine (CHPO 113B)

### (selon le mode opératoire général (C))

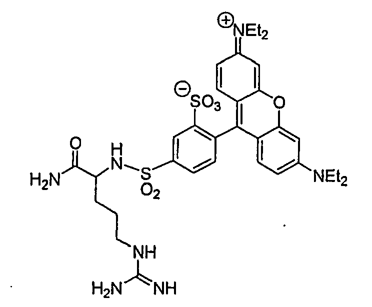

Dans une seringue Supelco, la résine Rink-Arg(pmc)-lissamine CHPO 113A (20 mg ; 0,048 mmol ; 0,416 mmol/g) est traitée avec 2 mL de TFA/DCM/H₂O (50:45:5) pendant 3 heures à température ambiante. La résine est filtrée, lavée avec du DCM (3 × 3 mL). Le filtrat est collecté et amené à sec sous pression réduite pour fournir un solide violet intense.

HPLC 80% (dionex, tr = 24.47 min ; λ = 288 nm ; gradient t : 0 min : 2% de CH₃CN dans H₂O (0.1% TFA) t = 30 min : 100% CH₃CN t = 40 min : 100% ; 0,5 mL/min). MS (ESI-TOF) *m*/*z* (M+H) calculé pour C₃₃H₄₃N₇O₇S₂ + H, 714 ; trouvé, 714. RMN ¹H (CD₃OD) δ 8,64-8,66 (m, 1H) ; 8,14-8,17 (m, 1H) ; 7,52-7,56 (m, 1H) ; 6,97-7,56 (m, 6H) ; 3,93-3,99 (m, 1H) ; 3,76-3,79 (m, 8H) ; 3,23-3,26 (m, 2H) ; 1,65-1,80 (m, 4H) ; 2,02-2,11 (m, 12H).

### II - PRÉPARATION DE CATIONS FLUORESCENTS

### Synthèse de la résine Rink-Phe-NHFmoc (CHPO 72A ou 116A)

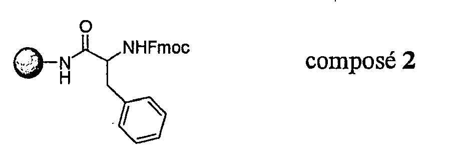

Dans un ballon, la résine Rink-NHFmoc (5 g ; 3 mmol ; 0,6 mmol/g)(composé 1) est traitée avec 50 mL d'une solution de 20% de pipéridine dans le DMF et agitée pendant 2 heures. Le mélange est filtré sur fritté et la résine est lavée avec deux séquences de 50 mL de DMF, DCM, MeOH et une séquence de 50 mL de DMF, DCM (résine CHPO 24A).

Une solution d'acide aminé préactivé est préparée en mélangeant dans l'ordre N-α-Fmoc-L-phénylalanine (1,068 g ; 2,76 mmol) et HOBt (422 mg ; 2,76 mmol) dans 20 mL de DMF/DCM (1:1), pendant 15 minutes à température ambiante. Le DIC (435 µL ; 2,76 mmol) est ajouté à la solution d'acide carboxylique et de HOBt et le mélange est agité pendant 15 minutes à température ambiante. Pendant ce temps, la résine déprotégée Rink-NH₂ CHPO 24A (2,0 g ; 1,38 mmol ; 0,69 mmol/g) est expansée avec 20 mL de DMF/DCM (1:4) dans un ballon, pendant 30 minutes. La solution d'acide carboxylique préactivée (2,76 mmol ; 2 éq.) est ajoutée à la résine. L'ensemble est agité pendant 14 heures à température ambiante. La résine est filtrée sur un fritté, lavée avec deux séquences de 20 mL de DMF, DCM, MeOH et une séquence de 20 mL de DMF, DCM, puis séchée sous pression réduite pendant 4 heures.

### Synthèse de H₂N-CO-Phe-NHFmoc (CHPO 116C)

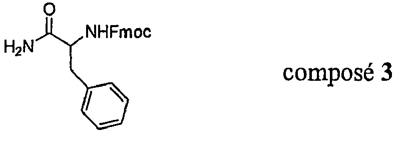

Dans une seringue Supelco, la résine Rink-Phe-NHFmoc CHPO 116A (composé 2)(80 mg ; 0,145 mmol ; 0,55 mmol/g) est traitée avec 1 mL de TFA/DCM/H₂O (20:75:5) pendant 2 heures à température ambiante. La résine est filtrée, lavée avec du DCM (3 × 3 mL). Le filtrat est collecté et amené à sec sous pression réduite pour fournir 11 mg de solide blanc.

Rdt = 64%. HPLC 99% (dionex, tr = 30.11 min ; λ = 288 nm ; gradient t : 0 min : 2% de CH₃CN dans H₂O (0.1% TFA) t = 30 min : 100% CH₃CN t = 40 min : 100% ; 0,5 mL/min). MS (ESI-TOF) *m*/*z* (M+H) calculé pour C₂₄H₂₂N₂O₃ + H, 387 ; trouvé, 387. RMN ¹H (CDCl₃) δ 7,78 (d ; J = 7,5 Hz ; 2H) ; 7,55 (dd ; J = 7,2 Hz ; J = 4,9 Hz ; 2H) ; 7,42 (dd ; J = 7,5 Hz ; J = 7,1 Hz ; 2H) ; 7,24-7,35 (m, 7H) ; 5,29-5,31 (m, 2H) ; 4,43-4,45 (m, 3H) ; 4,20 (t ; J = 6,4Hz ; 1H) ; 3,04-3,12 (m, 2H).

### Synthèse de la résine Rink-Phe-Lys(boc)-NHFmoc (CHPO 120A)

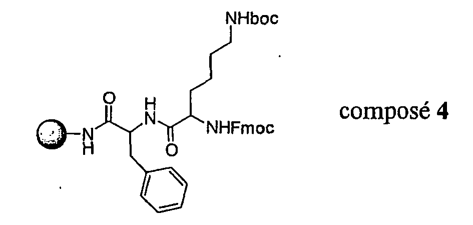

Dans un ballon, la résine Rink-Phe-NHFmoc CHPO 116A (composé 2)(1,1 g ; 0,605 mmol ; 0,55 mmol/g) est traitée avec 10 mL d'une solution de 20% de pipéridine dans le DMF et agitée pendant 2 heures. Le mélange est filtré sur fritté et la résine est lavée avec deux séquences de 10 mL de DMF, DCM, MeOH et une séquence de 10 mL de DMF, DCM (résine CHPO 118A).

Une solution d'acide aminé préactivé est préparée en mélangeant dans l'ordre N-α-Fmoc-N-ω-*te*rbutyloxycarbonyl-L-lysine (503 mg ; 1,075 mmol) et HOBt (164 mg ; 1,075 mmol) dans 5 mL de DMF/DCM (1:1), pendant 15 minutes à température ambiante. Le DIC (170 µL ; 1,075 mmol) est ajouté à la solution d'acide carboxylique et de HOBt et le mélange est agité pendant 15 min à température ambiante. Pendant ce temps, la résine déprotégée Rink-Phe-NH₂ CHPO 118A (857 mg ; 0,537 mmol ; 0,627 mmol/g) est expansée avec 10 mL de DMF/DCM (1:4) dans un ballon, pendant 30 min. La solution d'acide carboxylique préactivée (1,075 mmol ; 2 éq.) est ajoutée à la résine. L'ensemble est agité pendant 14 h à température ambiante. La résine est filtrée sur un fritté, lavée avec deux séquences de 10 mL de DMF, DCM, MeOH et une séquence de 10 mL de DMF, DCM, puis séchée sous pression réduite pendant 4 h.

### Synthèse de H₂N-CO-Phe-Lys-NHFmoc (CHPO 120C)

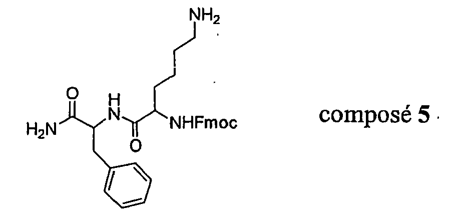

Dans une seringue Supelco, la résine Rink-Phe-Lys(boc)-NHFmoc CHPO 120A (composé **4**)(80 mg ; 0,163 mmol ; 0,49 mmol/g) est traitée avec 1 mL de TFA/DCM/H₂O (20:75:5) pendant 1 h à température ambiante. La résine est filtrée, lavée avec du DCM (3 × 3 mL). Le filtrat est collecté et amené à sec sous pression réduite pour fournir 13 mg de solide blanc.

Rdt = 65%. HPLC 97% (dionex, tr = 25.93 min ; λ = 288 nm ; gradient t : 0 min : 2% de CH₃CN dans H₂O (0,1% TFA) t = 30 min : 100% CH₃CN t = 40 min : 100% ; 0,5 mL/min). MS (ESI-TOF) *m*/*z* (M+H) calculé pour C₃₀H₃₄N₄O₄ + H, 515 ; trouvé, 515. RMN ¹H (CD₃OD) δ 7,78 (d ; J = 7,5 Hz ; 2H) ; 7,64-7,66 (m, 2H) ; 7,41 (dd ; J = 7.2 Hz ; J = 7,1 Hz ; 2H) ; 7,32 (dd ; J = 7,2 Hz ; J = 6,8 Hz ; 2H) ; 7,15-7,22 (m, 5H) ; 4,63-4,65 (m, 1H) ; 4,38-4,41 (m, 2H) ; 4,21-4,22 (m, 1H) ; 4,01-4,02 (m, 1H) ; 2,84-3,17 (m, 4H) ; 1,55-1,65 (m, 4H) ; 1,27-1,34 (m, 2H). RMN ¹³C (CD₃OD) δ 189,03 ; 157,63: (CO) ; 141,60 ; 137,18 : C quat, arom,, 131,40 ; 129,33 ; 128,41 ; 127,18 ; 126,74 ; 125,15 ; 119,96 : CH arom, 64,35 ; 55,32 ; 54,43 : CH, 66,98 ; 39,41 ; 37,71 ; 31,24 ; 26,99 ; 22,47 : CH₂.

### Synthèse de la résine Rink-Phe-Lys(boc)-Gly-NHFmoc (CHPO 125A)

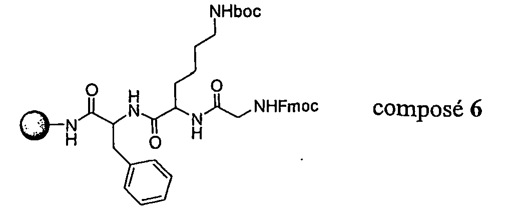

Dans une seringue Supelco, la résine Rink-Phe-Lys(boc)-NHFmoc CHPO 120A (composé **4**)(970 mg ; 0,475 mmol ; 0,49 mmol/g) est traitée avec 10 mL d'une solution de 20% de pipéridine dans le DMF et agitée pendant 2 h. Le mélange est filtré sur fritté et la résine est lavée avec deux séquences de 10 mL de DMF, DCM, MeOH et une séquence de 10 mL de DMF, DCM (résine CHPO 122A).

Une solution d'acide aminé préactivé est préparée en mélangeant dans l'ordre la N-α-Fmoc-Glycine (261 mg ; 0,88 mmol) et HOBt (134 mg ; 0,88 mmol) dans 5 mL de DMF/DCM (1:1), pendant 15 min à température ambiante. Le DIC (138 µL ; 0,88 mmol) est ajouté à la solution d'acide carboxylique et de HOBt et le mélange est agité pendant 15 min à température ambiante. Pendant ce temps, la résine déprotégée Rink-Phe-Lys(boc)-NH₂ CHPO 122A (800 mg ; 0,44 mmol ; 0,55 mmol/g) est expansée avec 5 mL de DMF/DCM (1:4) dans une seringue Supelco, pendant 30 min. La solution d'acide carboxylique préactivée (0,88 mmol ; 2 éq.) est ajoutée à la résine. L'ensemble est agité par rotation pendant 14 h à température ambiante. La résine est filtrée, lavée avec deux séquences de 10 mL de DMF, DCM, MeOH et une séquence de 10 mL de DMF, DCM, puis séchée sous pression réduite pendant 4 h.

### Synthèse de H₂N-CO-Phe-Lys-Gly-NHFmoc (CHPO 125B)

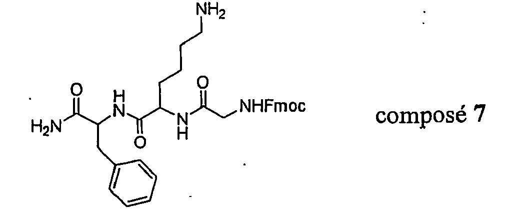

Dans une seringue Supelco, la résine Rink-Phe-Lys(boc)-Gly-NHFmoc CHPO 125A (composé 6)(100 mg ; 0,210 mmol ; 0,477 mmol/g) est traitée avec 1 mL de TFA/DCM/H₂O (20:75:5) pendant 1 h à température ambiante. La résine est filtrée, lavée avec du DCM (3 × 3 mL). Le filtrant est collecté et amené à sec sous pression réduite pour fournir 17 mg de solide blanc.

Rdt = 63%. HPLC 99% (dionex, tr = 25.54 min ; λ = 288 nm ; gradient t : 0 min : 2% de CH₃CN dans H₂O (0,1% TFA) t = 30 mien : 100% CH₃CN t = 40 min : 100% ; 0,5 mL/min). MS (ESI-TOF) *m*/*z* (M+H) calculé pour C₃₂H₃₇N₅O₅ + H, 572 ; trouvé, 572. RMN ¹H (CD₃OD) δ 7,80 (d ; J = 7,2 Hz ; 2H) ; 7,65 (d ; J = 7,2 Hz ; 2H) ; 7,40 (dd ; J = 7,2 Hz ; J = 7,1 Hz ; 2H) ; 7,15-7,34 (m, 7H) ; 4,59-4,62 (m, 1H) ; 4,37-4,39 (m, 2H) ; 4,24-4,25 (m, 2H) ; 3,74-3,78 (m, 2H) ; 3,22-3,30 (m, 1H) ; 2,95-3,00 (m, 1H) ; 2,80-2,83 (m, 2H) ; 1,50-1,60 (m, 4H) ; 1,27-1,31 (m, 2H). RMN ¹³C (CD₃OD) δ 172,61 ; 171,99 : (CO) ; 150,14 ; 144,13 ; 137,66 : C quat, arom, ; 129,26 ; 128,45 ; 127,83 ; 127,18 ; 126,74; 125,18 ; 119,96: CH arom, 54,77; 53,75 ; 47,30: CH; 67,34 ; 43,98 ; 39,40 ; 37,49 ; 34,94 ; 30,81 ; 26,89 ; 22,30 : CH₂.

### Synthèse de la résine Rink-Phe-Lys(boc)-Gly-Orn(boc)-NHFmoc (CHPO 126A)

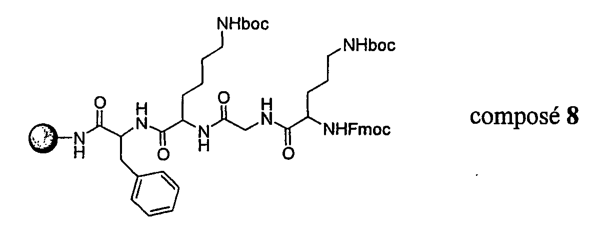

Dans une seringue Supelco, la résine Rink-Phe-Lys(boc)-Gly-NHFmoc CHPO 125A (composé 6)(740 mg ; 0,353 mmol ; 0,477 mmol/g) est traitée avec 5 mL d'une solution de 20% de pipéridine dans le DMF et agitée pendant 2 h. Le mélange est filtré sur fritté et la résine est lavée avec deux séquences de 5 mL de DMF, DCM, MeOH et une séquence de 5 mL de DMF, DCM (résine CHPO 125C).

Une solution d'acide aminé préactivé est préparée en mélangeant dans l'ordre la N-α-Fmoc-N-ω-*te*rbutyloxycarbonyl-L-ornithine (281 mg ; 0,619 mmol) et HOBt (83 mg ; 0,619 mmol) dans 3 mL de DMF/DCM (1:1), pendant 15 min à TA. Le DIC (98 µL ; 0,619 mmol) est ajouté à la solution d'acide carboxylique et de HOBt et le mélange est agité pendant 15 min à température ambiante. Pendant ce temps, la résine déprotégée Rink-Phe-Lys(boc)-Gly-NH₂ CHPO 125C (580 mg ; 0,309 mmol ; 0,534 mmol/g) est expansée avec 2 mL de DMF/DCM (1:4) dans une seringue Supelco, pendant 30 min. La solution d'acide carboxylique préactivée (0,88 mmol ; 2 éq.) est ajoutée à la résine. L'ensemble est agité par rotation pendant 14 h à température ambiante. La résine est filtrée, lavée avec deux séquences de 5 mL de DMF, DCM, MeOH et une séquence de 5 mL de DMF, DCM, puis séchée sous pression réduite pendant 4 h.

### Synthèse de H₂N-CO-Phe-Lys-Gly-Orn-NHFmoc (CHPO 126B)

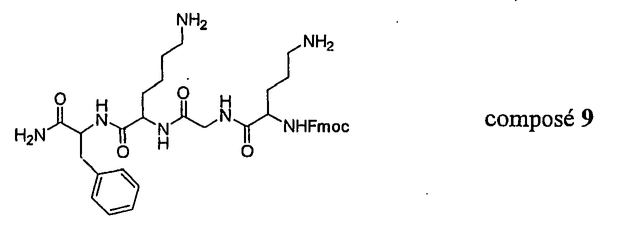

Dans une seringue Supelco, la résine Rink-Phe-Lys(boc)-Gly-Orn(boc)-NHFmoc CHPO 126A (composé 8)(80 mg ; 0,184 mmol ; 0,433 mmol/g) est traitée avec 1 mL de TFA/DCM/H₂O (20:75:5) pendant 1 h à température ambiante. La résine est filtrée, lavée avec du DCM (3 × 3 mL). Le filtrat est collecté et amené à sec sous pression réduite pour fournir 18 mg de solide blanc.

Rdt = 78%. HPLC 97% (dionex, tr = 23,37 min ; λ = 288 nm ; gradient t : 0 min : 2% de CH₃CN dans H₂O (0,1% TFA) t = 30 min : 100% CH₃CN t = 40 min : 100% ; 0,5 mL/min). MS (ESI-TOF) *m*/*z* (M+H) calculé pour C₃₇H₄₇N₇O₆ + H, 686 ; trouvé, 686. RMN ¹H (CD₃OD) δ 7,83 (d ; J = 7,2 Hz ; 2H) ; 7,69 (d ; J = 7,2 Hz ; 2H) ; 7,15-7,44 (m, 9H) ; 4,60-4,64 (m, 1H) ; 4,43-4,46 (m, 2H) ; 4,24-4,28 (m, 2H) ; 4,10-4,15 (m, 1H) ; 3,86-3,92 (m, 2H) ; 3,20-3,30 (m, 1H) ; 2,90-3,00 (m, 2H) ; 2,80-2,85 (m, 2H) ; 1,50-1,90 (m, 8H) ; 1,27-1,40 (m, 6H). RMN ¹³C (CD₃OD) δ 189,87 ; 175,06 ; 174,19 ; 172,72 ; 171,24 ; 157,71 : (CO) ; 144,20 ; 141,63 ; 137,67 : C quat, arom, ; 129,34 ; 128,43 ; 127,87 ; 127,21 ; 126,73 ; 125,17 ; 119,99 : CH arom, 55,18 ; 54,78 ; 53,97 ; 48,86 : CH ; 67,16 ; 44,70 ; 39,37 ; 37,53 ; 30,7 ; 29,72 ; 28,95 ; 26,84 ; 24,02 ; 22,36 : CH₂.

### Synthèse de la résine Rink-Phe-Lys(boc)-Gly-Orn(boc)-Bodipy (CHPO 129A)

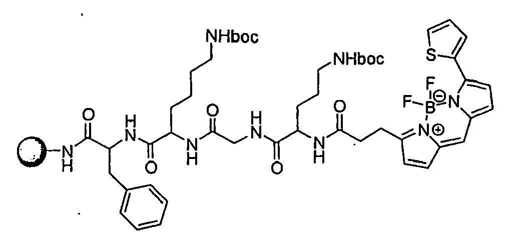

Dans une seringue Supelco, la résine Rink-Phe-Lys(boc)-Gly-Orn(boc)-NHFmoc CHPO 126A (composé 8)(625 mg ; 0,27 mmol ; 0,433 mmol/g) est traitée avec 5 mL d'une solution de 20% de pipéridine dans le DMF et agitée pendant 2 h. Le mélange est filtré sur fritté et la résine est lavée avec deux séquences de 5 mL de DMF, DCM, MeOH et une séquence de 5 mL de DMF, DCM (résine CHPO 127A).

Une solution d'acide carboxylique préactivé est préparée en mélangeant dans l'ordre l'acide 4,4-difluoro-5-(2-thiényl)-4-bora-3a,4a-diaza-*s*-indacène-3-propionique CHPO65C (20 mg ; 0,0576 mmol) et HOBt (10 mg ; 0,0768 mmol) dans 3 mL de DMF/DCM (1:1), pendant 15 min à température ambiante. Le DIC (12 µL ; 0,0768 mmol) est ajouté à la solution d'acide carboxylique et de HOBt et le mélange est agité pendant 15 min à température ambiante. Pendant ce temps, la résine déprotégée Rink-Phe-Lys(boc)-Gly-Orn(boc)-NH₂ CHPO 127A (80 mg ; 0,0384 mmol ; 0,48 mmol/g) est expansée avec 2 mL de DMF/DCM (1:4) dans une seringue Supelco, pendant 30 min. La solution d'acide carboxylique préactivée (0,0768 mmol ; 2 éq.) est ajoutée à la résine. L'ensemble est agité par rotation pendant 14 h à température ambiante. La résine est filtrée, lavée avec deux séquences de 5 mL de DMF, DCM, MeOH et une séquence de 5 mL de DMF, DCM, puis séchée sous pression réduite pendant 4 h.

### Synthèse de H₂N-CO-Phe-Lys-Gly-Orn-Bodipy (CHPO 129B)

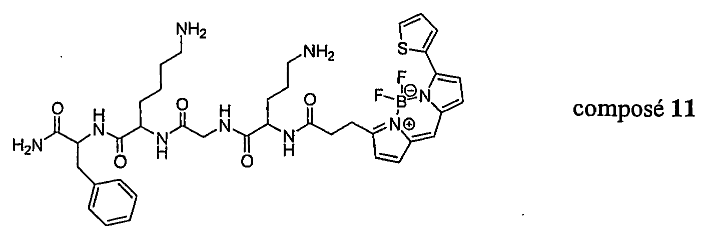

Dans une seringue Supelco, la résine Rink-Phe-Lys(boc)-Gly-Orn(boc)-Bodipy CHPO 129A (92 mg; 0,0384 mmol; 0,415 mmol/g) est traitée avec 1 mL de TFA/DCM/H₂O (20:75:5) pendant 1 h à température ambiante. La résine est filtrée, lavée avec du DCM (3 × 3 mL). Le filtrat est collecté et amené à sec sous pression réduite pour fournir 14 mg de solide violet intense.

Rdt = 53%. HPLC 86% (dionex, tr = 23,58 min ; λ = 288 nm ; gradient t : 0 min : 2% de CH₃CN dans H₂O (0,1% TFA) t = 30 min : 100% CH₃CN t = 40 min : 100% ; 0,5 mL/min). MS (ESI-TOF) *m*/*z* (M+H) calculé pour C₃₈H₄₈BF₂N₉O₅S + H, 792 ; trouvé, 792. RMN ¹H (CD₃OD) δ 8,11 (d ; J = 3,8 Hz ; 1H) ; 7,66 (d ; J = 4,9 Hz ; 1H) ; 7,47 (s, 1H) ; 7,16-7,29 (m, 8H) ; 6,90 (d ; J = 4,5 Hz ; 1H) ; 6,52 (d ; J = 3,8 Hz ; 1H) ; 4,59-4,63 (m, 1H) ; 4,29-4,21 (m, 2H) ; 3,77-4,00 (m, 2H) ; 3,20-3,25 (m, 2H) ; 2,94-2,99 (m, 4H) ; 2,77-2,81 (m, 4H) ; 1,30-1,90 (m, 10H).

Le composé 11 ainsi obtenu est un composé de formule (I-bis).

### Synthèse de la résine Rink-Phe-Lys(boc)-Gly-Orn(boc)-Lissamine (CHPO 130A)

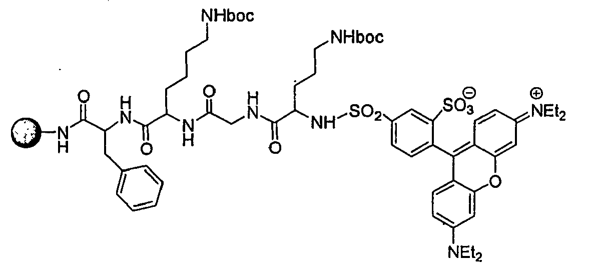

Dans une seringue Supelco, la résine Rink-Phe-Lys(boc)-Gly-Orn(boc)-NHFmoc CHPO 126A (composé 8)(625 mg ; 0,27 mmol ; 0,433 mmol/g) est traitée avec 5 mL d'une solution de 20% de pipéridine dans le DMF et agitée pendant 2 h. Le mélange est filtré sur fritté et la résine est lavée avec deux séquences de 5 mL de DMF, DCM, MeOH et une séquence de 5 mL de DMF, DCM (résine CHPO 127A).

Dans une seringue Supelco munie d'un fritté, la résine Rink-Phe-Lys(boc)-Gly-Orn(boc)-NH₂ CHPO127A (80 mg ; 0,0384 mmol ; 0,48 mmol/g) est expansée avec 1 mL de DCM/DMF (3:1). La DIEA (13 µL ; 0,0768 mmol ; 2 éq.) est ajoutée, puis la Lissamine rhodamine B sulfonyl chloride (44 mg ; 0,078 mmol ; 2 éq.) dissoute dans 3 mL de DCM/DMF (1:1). Le mélange réactionnel est agité 5 h à température ambiante. La résine est filtrée, lavée avec deux séquences de 3 mL de DMF, DCM, MeOH et une séquence de 3 mL de DMF, DCM, puis séchée sous pression réduite pendant 4 h.

### Synthèse de H₂N-CO-Phe-Lys-Gly-Orn-Lissamine (CHPO 130B)

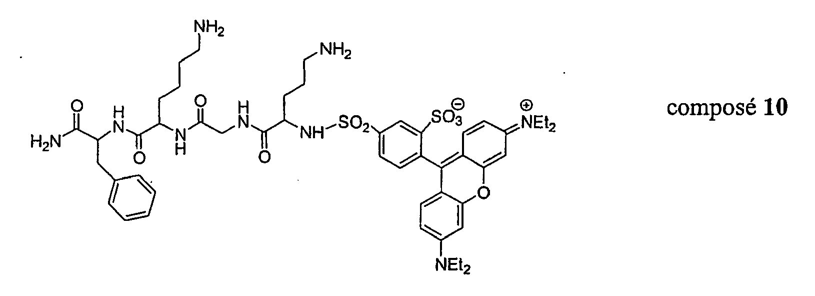

Dans une seringue Supelco, la résine Rink-Phe-Lys(boc)-Gly-Orn(boc)-lissamine CHPO 130A (100 mg; 0,0384 mmol; 0,38 mmol/g) est traitée avec 1 mL de TFA/DCM/H₂O (20:75:5) pendant 1 h à température ambiante. La résine est filtrée, lavée avec du DCM (3 × 3 mL). Le filtrat est collecté et amené à sec sous pression réduite pour fournir 17 mg de solide violet intense.

Rdt = 53%. HPLC 83% (dionex, tr = 23,76 min ; λ = 288 nm ; gradient t : 0 min : 2% de CH₃CN dans H₂O (0,1% TFA) t = 30 min : 100% CH₃CN t = 40 min : 100% ; 0,5 mL/min). MS (ESI-TOF) *m*/*z* (M+H) calculé pour C₄₉H₆₅N₉O₁₀S₂ + H, 1004 ; trouvé, 1004. RMN ¹H (CD₃OD) δ 8,64 (s, 1H) ; 8,18 (d ; J = 7,9 Hz ; 1H) ; 7,55 (d ; J = 7,9 Hz ; 1H) ; 6,97-7,28 (m, 11H) ; 4,61-4,65 (m, 1H) ; 4,12-4,14 (m, 1H) ; 3,96-4,00 (m, 1H) ; 3,67-3,70 (m, 8H) ; 3,20-3,30 (m, 1H) ; 2,95-3,05 (m, 4H) ; 2,82-2,90 (m, 4H); 1,21-1,86 (m, 22H). RMN ¹³C (CD₃OD) δ 174,89; 172,70 ; 170,40; 169,47 : (CO); 161,48 ; 158,40; 156,37; 156,28; 156,13 ; 145,76; 142,54; 137,76; 137,46; 134,83 ; 114,27 : C quat, arom, ; 132,78 ; 131,83 ; 130,01 ; 129,47 ; 129,37 ; 128,72 ; 128,37 ; 126,78 ; 126,56 ; 96,04 : CH arom, 56,69 ; 54,56 ; 52,80 : CH ; 45,80 ; 43,21 ; 39,40 ; 37,89 ; 31,15 ; 30,76 ; 29,74 ; 28,33 ; 26,98 ; 24,02 ; 22,91 ; 22,47 : CH₂; 11,84 : CH₃.

Le composé 10 ainsi obtenu est un composé de formule (I-bis).

### Autre exemple :

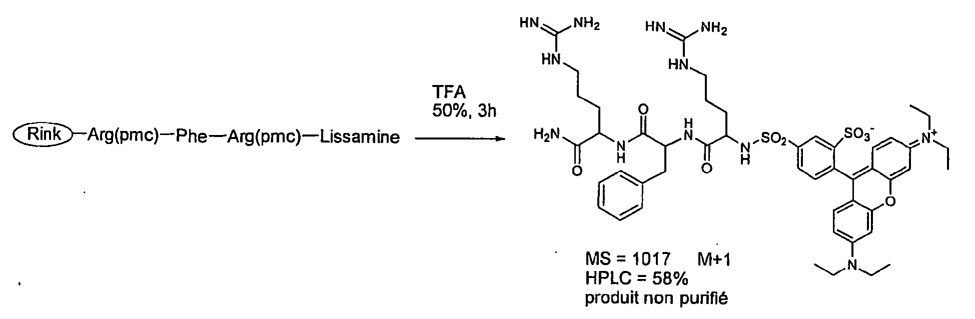

### Synthèse de la résine Rink-Arg(pmc)-Phe-Arg(pmc)-Lissamine (CHPO 133A)

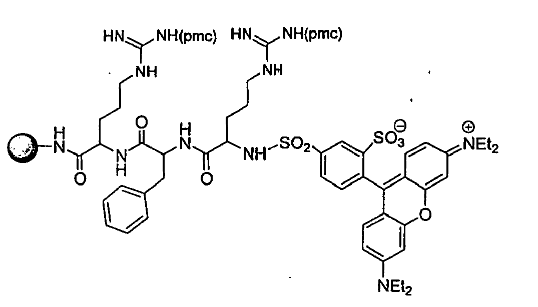

Dans une seringue Supelco, la résine Rink-Arg(pmc)-Phe-Arg(pmc)-NHFmoc CHPO 84A (803 mg ; 0,317 mmol ; 0,376 mmol/g) est traitée avec 5 mL d'une solution de 20% de pipéridine dans le DMF et agitée pendant 2 h. Le mélange est filtré sur fritté et la résine est lavée avec deux séquences de 5 mL de DMF, DCM, MeOH et une séquence de 5 mL de DMF, DCM (résine CHPO 84B).

Dans une seringue Supelco munie d'un fritté, la résine Rink-Arg(pmc)-Phe-Arg(pmc)-NH₂ CHPO84B (100 mg ; 0,041 mmol ; 0,41 mmol/g) est expansée avec 1 mL de DCM/DMF (3:1). La DIEA (14 µL ; 0,082 mmol ; 2 éq.) est ajoutée, puis la Lissamine rhodamine B sulfonyl chloride (47 mg ; 0,082 mmol ; 2 éq.) dissoute dans 3 mL de DCM/DMF (1:1). Le mélange réactionnel est agité 5 h à température ambiante. La résine est filtrée, lavée avec deux séquences de 3 mL de DMF, DCM, MeOH et une séquence de 3 mL de DMF, DCM, puis séchée sous pression réduite pendant 4 h.

### Synthèse de H₂N-CO-Arg-Phe-Arg-Lissamine (CHPO 133B)

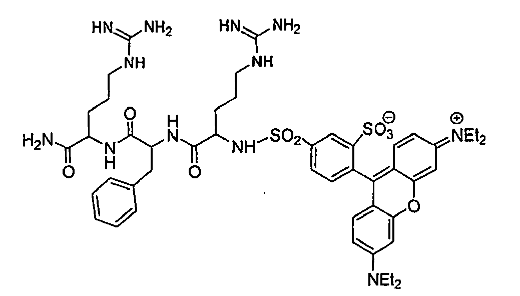

Dans une seringue Supelco, la résine Rink-Arg(pmc)-Phe-Arg(pmc)-lissamine CHPO 133A (122 mg; 0,041 mmol ; 0,336 mmol/g) est traitée avec 1 mL de TFA/DCM/H₂O (50:45:5) pendant 3 h à température ambiante. La résine est filtrée, lavée avec du DCM (3 × 3 mL). Le filtrat est collecté et amené à sec sous pression réduite pour fournir un solide violet intense.

HPLC 58% (dionex, tr = 24.29 min ; λ = 288 nm ; gradient t : 0 min : 2% de CH₃CN dans H₂O (0,1% TFA) t = 30 min : 100% CH₃CN t = 40 min : 100% ; 0,5 mL/min). MS (ESI-TOF) *m*/*z* (M+H) calculé pour C₄₈H₆₄N₁₂O₉S₂ + H, 1017 ; trouvé, 1017.

Le composé ainsi obtenu répond à la formule générale (III).

### III - MODES OPÉRATOIRES GÉNÉRAUX POUR LA SYNTHÈSE EN PLAQUES

### Mode opératoire général pour dispenser la résine dans une plaque de 96 puits (CHPO 190)

0,007 mmol × 96 puits × 1,1 éq. = 0,7392 mmol de résine Rink-NH₂ est placée dans un bécher et expansée avec 1 mL × 96 puits × 1,1 éq. = 105,6 mL de DCM/ DMF (1:1). La résine doit être parfaitement bien répartie dans le mélange de solvants. On distribue 1 mL de solution dans chaque puits, à l'aide d'une pipette multicanaux

### Mode opératoire général pour greffer un acide aminé sur la résine (CHPO 190)

Une solution d'acide aminé préactivé est préparée dans un pilulier en mélangeant dans l'ordre l'acide aminé-N-Fmoc protégé (0,021 mmol) dissous dans 0,2 mL de DMF et HOBt (0,021 mmol) dissous dans 0,2 mL de DMF, pendant 15 min à température ambiante. Le DIC (0,021 mmol) dilué dans 0,3 mL de DMF est ajouté à la solution d'acide carboxylique et de HOBt et le mélange est agité pendant 15 min à température ambiante. Pendant ce temps, la résine déprotégée-NH₂ (0,007 mmol) est expansée dans les puits avec 0,2 mL de DMF, qui est ensuite filtré. La solution d'acide carboxylique préactivée (0,7 mL ; 0,021 mmol ; 3 éq.) est ajoutée à la résine, dans chacun des puits, à l'aide d'une pipette multicanaux. On ajoute 0,3 mL de DMF pour compléter la réaction. Les plaques sont scellées grâce au système Flexchem et agitées pendant 14 h à température ambiante, sur le Robbin's. En fin de réaction, les plaques sont descellées, la résine est filtrée, lavée avec deux séquences de 0,6 mL de DMF, DCM, MeOH et une séquence de 0,6 mL de DMF, DCM.

### Mode opératoire général pour greffer un acide aminé sur la résine (CHPO 191)

La résine N-Fmoc-protégée (0,007 mmol) est expansée dans les puits avec 0,2 mL de DMF, qui est ensuite filtré. On ajoute ensuite dans chaque puits 1 mL d'une solution de 20% de pipéridine dans le DMF à l'aide d'une pipette multicanaux. Les plaques sont scellées grâce au système Flexchem et agitées pendant 3 h à température ambiante, sur le Robbin's. En fin de réaction, les plaques sont descellées, la résine est filtrée, lavée avec deux séquences de 0,6 mL de DMF, DCM, MeOH et une séquence de 0,6 mL de DMF, DCM.

### Mode opératoire général pour greffer la Lissamine (CHPO 198)

La résine (0,007 mmol) est expansée dans les puits avec 0,2 mL de DCM, qui est ensuite filtré. On ajoute dans chaque puits la DIEA (0,014 mmol) diluée avec 0,2 mL de DCM, puis la Lissamine-SO₂Cl (0,014 mmol) dissoute dans 0,6 mL de DCM, à l'aide d'une pipette multicanaux. Les puits sont complétés avec 0,2 mL de DCM. Les plaques sont scellées (Joints ChemTuff, adaptés au DCM) grâce au système Flexchem et agitées pendant 5 h à température ambiante, sur le Robbin's. En fin de réaction, les plaques sont descellées, la résine est filtrée, lavée avec DCM (2 × 0,6 mL), MeOH (1 × 0,6 mL) DMF (3 × 0,6 mL pendant 20 min) une séquence de 0,6 mL de DCM, MeOH, DMF, DCM, (DCM/DIEA 5%), DCM et enfin DMF (5 × 0,6 mL).

### Mode opératoire général pour le clivage (CHPO 199)

La résine (0,007 mmol) est expansée dans les puits avec 0,2 mL de DCM, qui est ensuite filtré. On ajoute dans chaque puits 0,6 mL d'une solution de TFA/DCM/H₂O (50:45:5), à l'aide d'une pipette multicanaux. Les plaques sont scellées grâce au système Flexchem et agitées pendant 3 h à température ambiante, sur le Robbin's. En fin de réaction, les plaques sont descellées et posées sur des plaques Deepwell pour récupérer le filtrat lors de la filtration de la résine. Celle-ci est filtrée, lavée avec du DCM (2 × 0,2 mL). Les plaques Deepwell sont amenées à sec à l'aide du Genevac DD4. La résine est à nouveau lavée avec du DMF (1 × 0,5 mL + 2 × 0,25 mL), puis les plaques sont amenées à sec à l'aide du Genevac.

### IV- EXEMPLES DE CRIBLAGE DES COLLECTIONS

### Exemple 1 : Expression du récepteur orphelin GPR 50 fusionné à son extrémité amino-terminale à la protéine EGFP et criblage de chimiothèque fluorescente.

### I) Fusion de la séquence codante du récepteur GPR 50 à la EGFP

La séquence codante du récepteur GPR50 (Genbank accession N°: U 52219) est placée dans le vecteur pCEP4-SP-EGFP. Ces extrémités 3' et 5' contiennent des séquences complémentaires aux extrémités 5' et 3' du vecteur pCEP4-SP-EGFP digéré par Fse1. Ces séquences complémentaires ont été introduites dans les amorces utilisées lors de la PCR (séquences soulignées) pour amplifier la séquence codante du récepteur.

Amorce sens : 5' G GCC GGG GCC GGG ACC CCC TAT GGC TGT ATT GGC

Amorce anti-sens : 5' CTC GTT CTC GTT GGA TCA CAC AGC CAT TTC ATC AGG ATC

Ce procédé d'insertion directionnelle de fragments de PCR dans un vecteur, ne fait pas intervenir d'enzyme de restriction (Aslanidis C et al ; 1990, Nucleic Acids Res.18, 6069-6074, Haun R.S. et al ; 1992, BioTechniques 13, 515-518).

Dans la construction pCEP4-SP-EGFP-GPR50, un bras espaceur long de 6 acides aminés sépare la EGFP du récepteur. Quant au récepteur, il possède une extrémité amino-terminale longue de 30 acides aminés, qui a été tronquée de 9 acides aminés par rapport à sa séquence sauvage.

### II) Expression des protéines recombinantes

Des cellules HEK 293 sont transfectées, par la méthode de la précipitation au phosphate de calcium (Chen & Okayama 1987, Mol.Cell.Biol.7 : 2745-2752) par la construction pCEP4-SP-EGFP-GPR50. Des lignées stables sont établies par sélection des cellules transfectées résistantes à l'hygromycine (500 µg/ml, Clontech). Les cellules sont cultivées dans un milieu MEM (Gibco) supplémenté de 10% de sérum de veau foetal (Sigma), de Pénicilline (100 unités/ml), streptomycine (100 µg/ml) et de glutamine (4 mM).

### III) Mesures de l'expression du récepteur GPR50 fusionné à la EGFP, par fluorimétrie

Les expériences de fluorescence sont effectuées dans une cuvette de 1ml pourvue d'un système d'agitation magnétique et placée dans un spectrofluorimètre Fluorolog (SPEX) équipé d'une lampe Xe 450 W (Osram) et de monochromateurs Spex 1680 0.22 m (excitation) et Spex 1681 0.22 m (émission). Les cellules sont mises en suspension dans un tampon physiologique : Hepes 10 mM, NaCl 137,5 mM, MgCl₂ 1,25 mM, CaCl₂ 1,25 mM, KCl 6 mM, glucose 5,6 mM, NaH₂PO₄ 0,4 mM, BSA 0,1 % (W/v), pH 7,4.

Les cellules entières sont récoltées après traitement à la trypsine-EDTA (1x Gibco) à température ambiante. Les cellules sont centrifugées 5 minutes à 100 g et sont remises en suspension à une concentration de 1 à 2.10⁶ cellules/ml dans le tampon physiologique.

Un spectre d'émission des cellules transfectées par le pCEP4-SP-EGFP-GPR50 est représenté dans la figure 1. Un spectre est réalisé avant chaque criblage. Les spectres font clairement apparaître le signal de la EGFP, indiquant que ces cellules expriment bien la EGFP en comparaison des cellules non transfectées.

### IV) Criblage de collections de composés fluorescents sur la lignée HEK exprimant le récepteur GPR50 fusionné à la EGFP

### IV.1. Procédure de criblage

La distribution des ligands et des cellules est réalisée à l'aide d'un robot pipetteur distributeur (Biomek 2000) et la fluorescence est mesurée dans un spectrofluorimètre (Victor II, Perkin Elmer).

Dans une plaque noire, 96 puits (Labsystem), sont distribués, chronologiquement, 98 µl de tampon physiologique, 2 à 4 µl de ligand fluorescent et 100 000 cellules (100 µl) d'une suspension cellulaire.

Les ligands fluorescents sont criblés à une concentration finale variant de 500 nM à 1 µM. Ces ligands sont dissous dans le DMSO, dont la concentration finale dans l'essai varie entre 1 et 2 %. Les cellules sont distribuées depuis un réservoir, dans lequel les cellules sont gardées en suspension par une alternance d'aspiration et refoulement effectuée par le robot avant chaque distribution. Afin de mélanger l'ensemble des composés, le robot aspire et refoule une fois, la moitié du puits. La plaque est ensuite incubée 10 min à température ambiante, puis centrifugée 5 min à 100g. Elle est ensuite placée dans le détecteur de fluorescence Victor. La EGFP est excitée à 465 ± 7 nm et son émission est mesurée à 510 ± 7 nm, pendant 2s, à 0.8 mm du fond de la plaque.

Dans une plaque 96 puits, 16 puits servent de contrôle et 80 ligands fluorescents sont testés, soit un par puits. L'émission de la EGFP (510 nm) de chacun des puits traités est comparée à la fluorescence moyenne de la EGFP (510 nm) des 16 puits contrôle. Un pourcentage d'extinction de fluorescence est ainsi calculé et correspond au critère d'identification des touches.

Trois mille ligands fluorescents ont été testés suivant ce protocole.

### IV.2. Confirmation et structure des touches potentielles

La liaison de certaines molécules a été vérifiée au spectrofluorimère. Les mesures sont effectuées dans une cuvette de 1 ml avec une suspension de 1 à 2.10⁶ cellules/ml de tampon physiologique.

Deux des ligands préférés découverts par criblage (voir Figure 2) possèdent les structures suivantes :

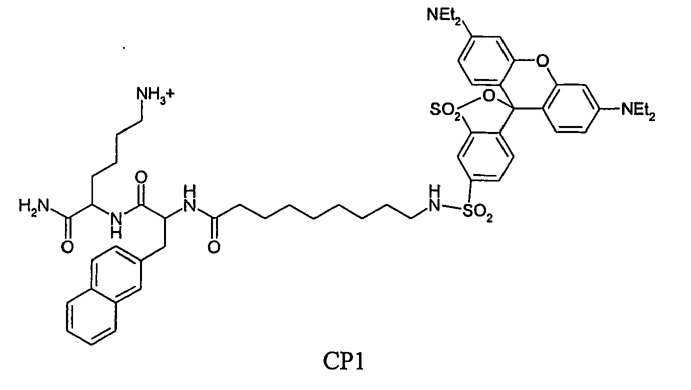

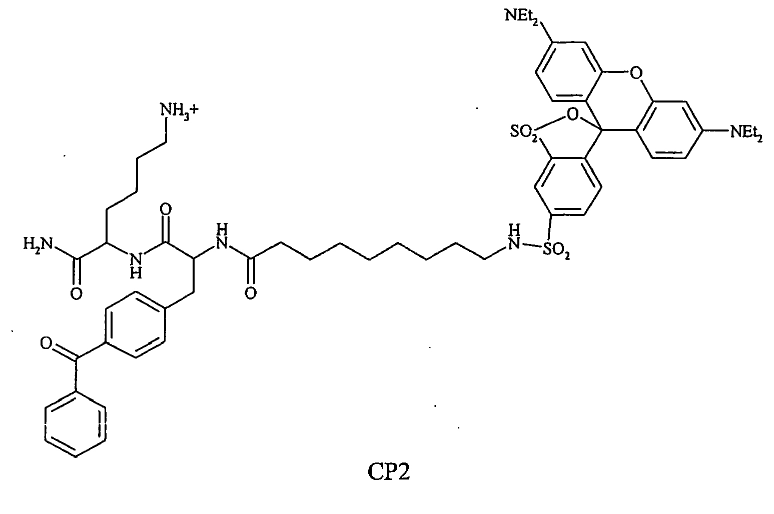

CP1 correspond à un composé de formule (I) susmentionnée dans laquelle n = 2, R₁ représente la chaîne latérale de la lysine, R₂ représente la chaîne latérale de la 2-naphtylalanine et A représente un groupe de la forme D-G, D représentant un groupe espaceur de formule -CO-(CH₂)₈-NH-SO₂- et A représentant un dérivé de lissamine.

CP2 correspond à un composé de formule (I) susmentionnée dans laquelle n = 2, R₁ représente la chaîne latérale de la lysine, R₂ représente la chaîne latérale de la phénylalanine de benzoyle et A représente un groupe de la forme D-G, D représentant un groupe espaceur de formule -CO-(CH₂)₈-NH-SO₂- et A représentant un dérivé de lissamine.

### Exemple 2 : Expression du récepteur mGluR8 fusionné à son extrémité amino-terminale à la protéine GFP et criblage de chimiothèques fluorescentes.

### I) Fusion de la séquence codante du récepteur mGluR8 à la GFP

La séquence codante du récepteur métabotrope glutamatergique sous type 8 (mGluR8) (Genbank accession N° : P70579) est placée dans le vecteur pcDNA6. Les extrémités 3' et 5' de la séquence codante contiennent des séquences complémentaires aux extrémités 5' et 3' du vecteur pcDNA6 digéré par Fse1. Ces séquences complémentaires ont été introduites dans les amorces utilisées lors de la PCR (séquences soulignées) pour amplifier la séquence codante du récepteur.

La séquence codante du récepteur est amplifiée par PCR en utilisant les amorces :
Amorces sens : 5' GGCCGGGGCCGGGAGCCCCTGGGCTGTGGTACCT
Amorce anti-sens : 5' CTCGTTCTCGTTGGATTAGATCGAATGATTACTGTAGCTG

Cette séquence est placée en aval de la séquence codante de la protéine GFP, elle-même placée en aval de la séquence codant pour le peptide signal de la sous-unité alpha 7 de poulet du récepteur nicotinique de l'acétylcholine (correspondant à la séquence en acide aminé 1 à 25 de la séquence de la protéine décrite dans la Genbank accession N° : X52295). La construction obtenue est applelée pcDNA6-SP-GFP-mGluR8.

Ce procédé d'insertion directionnelle de fragments de PCR dans un vecteur, ne fait pas intervenir d'enzyme de restriction (Aslanidis C et al; 1990, Nucleic Acids Res.18, 6069-6074, Haun R.S. et al ; 1992, BioTechniques 13, 515-518).

Dans la construction pcDNA6-SP-GFP-mGluR8, un bras espaceur long de 6 acides aminés sépare la GFP du récepteur. Quant au récepteur, son extrémité amino-terminale composée de 550 acides aminés a été tronquée de 547 acides aminés. Dans cette construction, il y a donc 9 acides aminés qui séparent la GFP du premier domaine transmembranaire du récepteur.

### II) Expression des protéines recombinantes

Des cellules HEK 293 sont transfectées par un agent de transfection la lipofectamine 2000 (Invitrogen) selon les instructions du fournisseur avec la construction pcDNA6-SP-GFP-mGluR8. Des lignées stables sont établies par sélection des cellules transfectées résistantes à la blasticidine (5 µg/ml, Invivogen). Les cellules sont cultivées dans un milieu MEM (PAA) contenant de la L-glutamine (2 mM) et supplémenté de 10% de sérum de veau foetal (PAA), de Pénicilline (100 unités/ml), streptomycine (100 µg/ml).

### III) Mesures de l'expression du récepteur mGluR8 fusionné à la GFP, par fluorimétrie

Les expériences de fluorescence sont effectuées dans une cuvette de 1 ml pourvue d'un système d'agitation magnétique et placée dans un spectrofluorimètre Fluorolog-3 (Jobin-Yvon Horiba). Les cellules sont mises en suspension dans un tampon physiologique : Hepes 10 mM ; NaCl 137,5 mM ; MgCl₂ 1,25 mM ; CaCl₂ 1,25 mM ; KCl 6 mM ; glucose 5,6 mM ; NaH₂PO₄ 0,4 mM ; BSA 0,1 % (W/v) pH 7,4.

Les cellules entières sont récoltées après traitement au PBS (PAA), EDTA 5 mM à température ambiante. Les cellules sont centrifugées 5 minutes à 100 g et sont remises en suspension à une concentration de 1 à 2.10⁶ cellules/ml dans le tampon physiologique.

Un spectre d'émission des cellules transfectées par le pcDNA-SP-GFP-mGluR8 est représenté dans la figure 3. Un spectre est réalisé avant chaque criblage. Les spectres font clairement apparaître le signal de la GFP, indiquant que ces cellules expriment bien la GFP en comparaison des cellules non transfectées.

### IV) Criblage de collections de composés fluorescents sur la lignée HEK exprimant le récepteur mGluR8 fusionné à la GFP

### IV.1. Procédure de criblage

Vingt quatre heures avant le criblage, les cellules sont ensemencées dans des plaques 96 puits. Au moment de l'expérience, les puits sont rincés et les cellules incubées dans un tampon physiologique dont la composition est décrite dans le paragraphe III. Les ligands fluorescents à tester sont distribués sur les cellules par la Flexstation (Molecular Devices) qui suit l'évolution de la fluorescence de la GFP (excitation 475 +/- 8 nm, émission 510 +/- 8 nm) au cours du temps.

Les ligands fluorescents sont criblés à une concentration finale variant de 150 nM à 500 nM. Ces ligands sont dissous dans le DMSO, dont la concentration finale dans l'essai varie entre 1 et 2 %. Un pourcentage d'extinction de fluorescence de la GFP est calculé pour chaque puits et correspond au critère d'identification des touches.

Environ 630 ligands fluorescents issus de la chimiothèque décrite dans la présente invention ont été testés suivant ce protocole.

### Exemple 3 : Expression du récepteur du Glucagon-like peptide-1 (GLP-1) fusionné à son extrémité amino-terminale à la protéine GFP et criblage de chimiothèque fluorescente.

### I) Fusion de la séquence codante du récepteur GLP-1 à la GFP

Un récepteur GLP-1R fluorescent est obtenu par fusion d'une partie de sa séquence codante à celle de la GFP. La séquence codante du récepteur GLP-1 (Genbank accession N°: NM_002062) est placée dans un vecteur pcDNA6 en aval de la séquence codante de la protéine autofluorescente GFP, elle-même placée en aval de la séquence codante pour le peptide signal de la sous-unité alpha 7 de poulet du récepteur nicotinique de l'acétylcholine (correspondant à la séquence en acide aminé 1 à 25 de la séquence de la protéine décrite dans la Genbank accession N° : NP_989512). Dans la construction ainsi obtenue, appelée pCDNA6-SP-GFP-GLP1R, un bras espaceur correspondant à la séquence codant pour deux acides aminés sépare la GFP du récepteur. Quant au récepteur, une version tronquée de 134 acides aminés dans sa partie amino-terminale a été utilisée pour cette étude. Cette variante du récepteur possède la caractéristique de ne plus permettre la liaison de son ligand endogène, le peptide apparenté au Glucagon, GLP-1.

### II) Expression des protéines recombinantes

Des cellules HEK 293 sont transfectées par un agent de transfection la lipofectamine 2000 (Invitrogen) selon les instructions du fournisseur avec la construction pcDNA6-SP-GFP-GLP-1R. Des lignées stables sont établies par sélection des cellules transfectées résistantes à la blasticidine (5 µg/ml, Invivogen). Les cellules sont cultivées dans un milieu MEM (PAA) contenant de la L-glutamine (2 mM) et supplémenté de 10% de sérum de veau foetal (PAA), de Pénicilline (100 unités/ml) et de streptomycine (100 µg/ml).

### III) Mesures de l'expression du récepteur GLP-1 fusionné à la GFP, par fluorimétrie

Les mesures de l'expression du récepteur GLP-1 fusionné à la GFP par fluorimétrie sont réalisées comme décrit précédemment dans l'exemple 2.

### IV) Criblage de collections de composés fluorescents sur la lignée HEK exprimant le récepteur GLP1R fusionné à la GFP

Les procédures de criblage et de confirmation des touches ont été réalisées comme précédemment décrit dans l'exemple 2.

Environ 630 ligands fluorescents issus de la chimiothèque décrite dans la présente invention ont été testés suivant ce protocole.

## Revendications

1. Utilisation d'une collection de composés traçables de type cationique répondant à la formule (I-1) suivante : 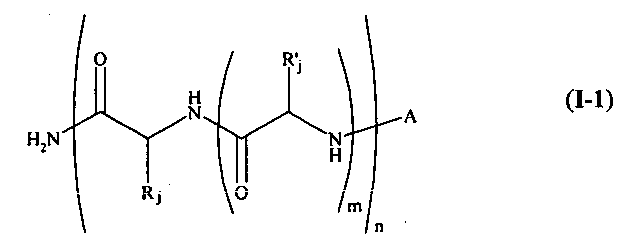 dans laquelle :
- m est égal à 0 ou 1,
- n représente un nombre entier variant de 1 à 10, et notamment de 1 à 6, et étant de préférence égal à 2 ou à 3,
- j représente un nombre entier variant de 1 à n,
- Rⱼ et R'ⱼ représentent une chaîne latérale d'acide aminé, l'un au moins des Rⱼ et R'ⱼ représentant une chaîne latérale d'acide aminé à caractère basique, et, lorsque m = 1, au moins deux des groupes Rⱼ représentent une chaîne latérale d'acide aminé à caractère basique et tous les R'ⱼ sont différents d'une chaîne latérale d'acide aminé à caractère basique,
- A représente un groupement traceur qui est une entité chimique fluorescente ou une entité chimique possédant des propriétés de colorant ou d'absorption de certaines longueurs d'onde, ou un groupe de la forme D-G, D représentant un groupe espaceur qui est une entité séparant le groupe traceur du reste de la molécule et G représentant un groupe traceur tel que défini précédemment,
pour la détermination *in vitro* de ligands d'un récepteur dont on ne connaît pas de ligand ou dont on ne connaît pas de ligand utilisable pour les études de liaison d'affinité spécifique.

2. Utilisation selon la revendication 1, **caractérisée en ce que** les composés traçables de type cationique répondent à la formule suivante : 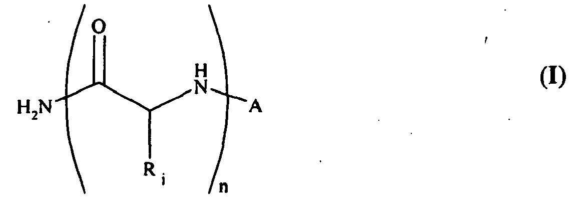 dans laquelle :
- n représente un nombre entier variant de 1 à 10, et notamment de 1 à 6, et étant de préférence égal à 2 ou à 3,
- i représente un nombre entier variant de 1 à n,
- Rᵢ représente une chaîne latérale d'acide aminé, l'un au moins des Rᵢ représentant une chaîne latérale d'acide aminé à caractère basique,
- A représente un groupement traceur tel que défini dans la revendication 1, ou un groupe de la forme D-G, D représentant un groupe espaceur tel que défini dans la revendication 1 et G représentant un groupe traceur tel que défini précédemment,
pour la détermination *in vitro* de ligands d'un récepteur dont on ne connaît pas de ligand ou dont on ne connaît pas de ligand utilisable pour les études de liaison d'affinité spécifique.

3. Utilisation selon l'une quelconque des revendications 1 ou 2, **caractérisée en ce que** le groupement traceur est un fluorophore, un colorant ou un "quencher".

4. Utilisation selon la revendication 1, **caractérisée en ce que** les composés traçables de type cationique répondent à la formule suivante : 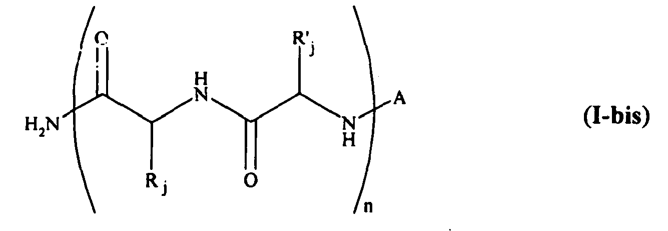 dans laquelle :
- n représente un nombre entier variant de 2 à 10, et notamment de 2 à 6, et étant de préférence égal à 2 ou à 3,
- j répond à la définition donnée dans la revendication 1,
- Rⱼ et R'ⱼ représentent une chaîne latérale d'acide aminé, **caractérisés en ce qu'**au moins deux des groupes Rⱼ représentent une chaîne latérale d'acide aminé à caractère basique et **en ce que** tous les R'ⱼ soient différents d'une chaîne latérale d'acide aminé à caractère basique, et
- A est tel que défini dans la revendication 1.

5. Utilisation selon l'une quelconque des revendications 1 à 4, **caractérisée en ce que** les composés traçables de type cationique sont utilisés sous forme de sels.

6. Utilisation selon l'une des revendications 1 à 5, pour la détermination *in vitro* de ligands d'un récepteur dont on ne connaît pas de ligand ou dont on ne connaît pas de ligand utilisable pour les études de liaison d'affinité spécifique.

7. Procédé de criblage de ligands de récepteurs dont on ne connaît pas de ligand ou dont on ne connaît pas de ligand utilisable, ledit procédé comprenant les étapes suivantes :
- l'utilisation selon l'une quelconque des revendications 1 à 5 impliquant la mise en présence d'une collection de composés traçables avec des cellules transfectées par une construction contenant la fusion de la séquence codant pour une protéine fluorescente, avec la séquence nucléotidique codant pour un récepteur dont on ne connaît pas de ligand ou dont on ne connaît pas de ligand utilisable, et le mélange desdites cellules et de ladite collection,
- la détection de la fluorescence dudit mélange, par excitation de ladite protéine fluorescente et mesure de la fluorescence d'émission de ladite protéine fluorescente, et la détermination du pourcentage d'extinction de fluorescence en comparant la fluorescence d'émission de ladite protéine fluorescente dans le mélange à la fluorescence moyenne de ladite protéine fluorescente en l'absence de ligand,
la fluorescence moyenne de ladite protéine fluorescente en absence de ligand étant mesurée par des essais témoins correspondant à la mesure de la fluorescence de la protéine fluorescente en l'absence de la collection de composés, et
- la détermination des composés qui donnent un pourcentage d'extinction de la fluorescence de la protéine fluorescente d'au moins 5% et leur identification en tant que ligand.

8. Procédé selon la revendication 7, **caractérisé en ce que** le récepteur est choisi parmi les récepteurs couplés aux protéines G (RCPG).

9. Procédé selon la revendication 7, **caractérisé en ce que** le récepteur est choisi parmi GPR50, GPR37, GPR19, GPR15, GPR31, GPR81, GPR3 et EDG7, le récepteur APJ, FPRL1, les récepteurs des chemokines CXCR1, CXCR2, CXCR3, CXCR4, les récepteurs des peptides Glucagon-like (GLP-1R et GLP-2R), les récepteurs CRF1 et CRF2, les récepteurs métabotropiques au glutamate (mGluR) et les récepteurs au GABA.

10. Procédé selon l'une quelconque des revendications 8 ou 9, **caractérisé en ce que** le récepteur est choisi parmi les récepteurs couplés aux protéines G orphelins.

## Claims

1. Use of a collection of traceable cationic compounds of the following formula (I-1): 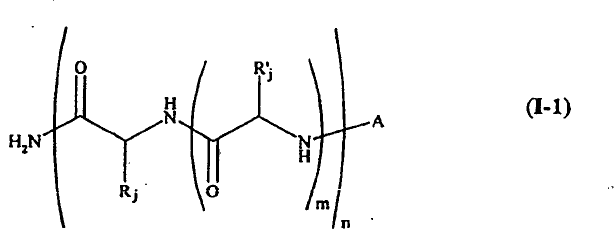 wherein:
- m is equal to 0 or 1,
- n represents an integer varying from 1 to 10, and in particular from 1 to 6, and being preferably equal to 2 or 3,
- j represents an integer varying from 1 to n,
- Rⱼ and R'ⱼ represent an amino acid side chain, at least one of Rⱼ and R'ⱼ representing a basic amino acid side chain, and, when m=1, at least two of the Rⱼ groups represent a basic amino acid side chain and all. R'ⱼ are different from a basic amino acid side chain,
- A represents a tracer group which is a fluorescent chemical entity or a chemical entity having the properties of staining or of absorbing certain wavelengths, or a group of form D-G, D representing a spacer group which is an entity separating the tracer group from the rest of the molecule and G representing a tracer group such as defined previously,
for the determination *in vitro* of ligands of a receptor for which a ligand is not known or for which a ligand usable for specific affinity binding studies is not known.

2. The use according to claim 1, **characterized in that** the traceable cationic compounds are of the following formula: 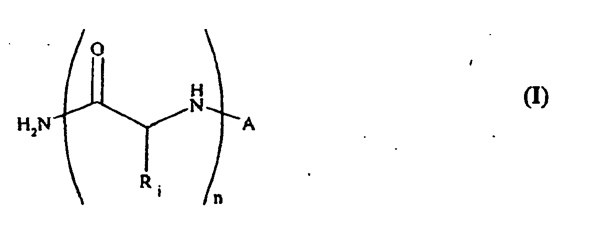 wherein:
- n represents an integer varying from 1 to 10, and in particular from 1 to 6, and being preferably equal to 2 or 3,
- i represents an integer varying from 1 to n,
- Rᵢ represents an amino acid side chain, at least one of Rᵢ representing a basic amino acid side chain,
- A represents a tracer group such as defined in claim 1, or a group of the form D-G, D representing a spacer group such as defined in claim 1 and G representing a tracer group such as defined previously,
for the determination *in vitro* of ligands of a receptor for which a ligand is not known or for which a ligand usable for specific affinity binding studies is not known.

3. The use according to either of claims 1 or 2, **characterized in that** the tracer group is a fluorophore, a stain or a quencher.

4. The use according to claim 1, **characterized in that** the traceable cationic compounds are of the following formula: 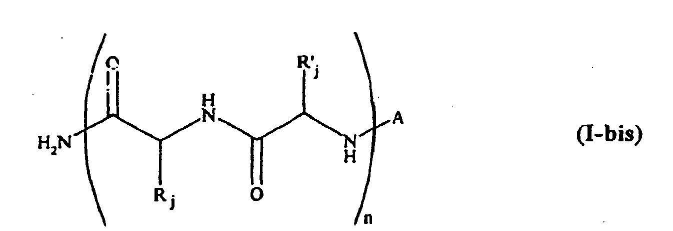 wherein:
- n represents an integer varying from 2 to 10, and in particular from 2 to 6, and being preferably equal to 2 or 3,
- j meets the definition given in claim 1,
- Rⱼ and R'ⱼ represent an amino acid side chain, **characterized in that** at least two of the Rj groups represent a basic amino acid side chain and **in that** all R'ⱼ are different from a basic amino acid side chain, and,
- A is such as defined in claim 1.

5. The use according to any of claims 1 to 4, **characterized in that** the traceable cationic compounds are used in salt form.

6. The use according to one of claims 1 to 5, for the determination *in vitro* of ligands of a receptor for which a ligand is not known or for which a ligand usable for specific affinity binding studies is not known.

7. A method for screening ligands for receptors for which a ligand is not known or for which a usable ligand is not known, said method comprising the following steps:
- the use according to any of claims 1 to 5 involving the placing, in the presence of a collection of traceable compounds, cells transfected by a construction containing the fusion of the sequence coding for a fluorescent protein with the nucleotide sequence coding for a receptor for which a ligand is not known or for which a usable ligand is not known, and the mixing of said cells and said collection,
- detection of the fluorescence of said mixture, by excitation of said fluorescent protein and measurement of the emission fluorescence of said fluorescent protein, and determination of the percentage of extinction of fluorescence by comparing the emission fluorescence of said fluorescent protein in the mixture with the mean fluorescence of said fluorescent protein in the absence of ligand,
the mean fluorescence of said fluorescent protein in the absence of ligand being measured by control studies corresponding to the measurement of the fluorescence of fluorescent protein in the absence of the collection of compounds, and,
- determination of the compounds that give a percentage of extinction of fluorescence of the fluorescent protein of at least 5% and their identification as a ligand.

8. The method according to claim 7, **characterized in that** the receptor is selected among the G protein coupled receptors (GPCR).

9. The method according to claim 7, **characterized in that** the receptor is selected among GPR50, GPR37, GPR19, GPR15, GPR31, GPR81, GPR3 and EDG7, APJ receptor, FPRL1, chemokine receptors CXCR1, CXCR2, CXCR3, CXCR4, the glucagon-like peptide receptors (GLP-1R and GLP-2R), CRF1 and CRF2 receptors, metabotropic glutamate receptors (mGluR) and GABA receptors.

10. The method according to either of claims 8 or 9, **characterized in that** the receptor is selected among the orphan G protein coupled receptors.

## Patentansprüche

1. Verwendung einer Kollektion nachvollziebarer Verbindungen kationischen Typs, die der folgenden Formel (I-1) entsprechen: 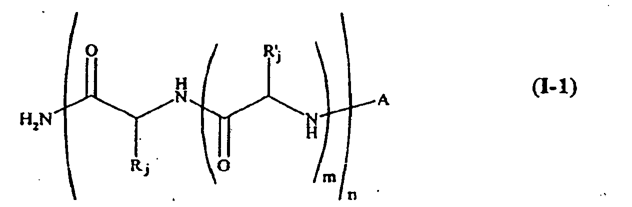 in welcher:
- m gleich 0 oder 1 ist,
- n eine ganze, von 1 bis 10 variierende Zahl darstellt, und vor allem von 1 bis 6, und die vorzugsweise gleich 2 oder 3 ist,
- j eine ganze, von 1 bis n variierende Zahl darstellt,
- Rⱼ und R'ⱼ eine seitliche Aminosäurenkette darstellen, wobei mindestens eines der Rⱼ und R'ⱼ eine seitliche Aminosäurenkette mit basischem Charakter darstellt, und, wenn m = 1, mindestens.zwei der Gruppen Rⱼ eine seitliche Aminosäurenkette mit basischem Charakter darstellen und alle R'ⱼ von einer seitlichen Aminosäurenkette mit basischem Charakter unterschiedlich sind,
- A eine Tracergruppe darstellt, die ein fluoreszierendes chemisches Gebilde ist oder ein chemisches Gebilde, das Farbstoff- oder Absorptionseigenschaften bestimmter Wellenlängen besitzt, oder eine Gruppe der Form D-G, wobei D eine Spacergruppe darstellt, die eine die Tracergruppe vom Rest des Moleküls trennende Einheit ist und G eine Tracergruppe darstellt, wie zuvor definiert,
für die *in vitro*-Determinierung von Liganden eines Rezeptors, von dem kein Ligand bekannt ist oder von dem kein Ligand bekannt ist, der für die Verbindungstests von spezifischer Affinität verwendbar ist.

2. Verwendung nach Anspruch 1, **dadurch gekennzeichnet, dass** die nachweisbaren Verbindungen kationischen Typs der folgenden Formel (I) entsprechen: 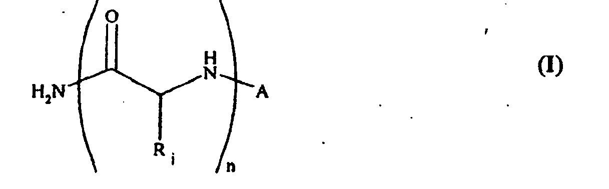 in welcher:
- n eine ganze, von 1 bis 10 variierende Zahl darstellt, und vor allem von 1 bis 6, und die vorzugsweise gleich 2 oder 3 ist,
- i eine ganze, von 1 bis n variierende Zahl darstellt,
- Rᵢ eine seitliche Aminosäurenkette darstellt, wobei mindestens eines der Rᵢ eine seitliche Aminosäurenkette mit basischem Charakter darstellt,
- A eine wie in Anspruch 1 definierte Tracergruppe darstellt oder eine Gruppe der Form D-G, wobei D eine wie in Anspruch 1 definierte Spacergruppe darstellt und G eine Tracergruppe darstellt, wie zuvor definiert,
für die *in vitro*-Determinierung von Liganden eines Rezeptors, von denen kein Ligand bekannt ist oder von denen kein Ligand bekannt ist, der für die Verbindungstest von spezifischer Affinität verwendbar ist.

3. Verwendung nach einem der Ansprüche 1 oder 2, **dadurch gekennzeichnet, dass** die Tracergruppe ein Fluorophor, ein Farbstoff oder ein Quencher ist.

4. Verwendung nach Anspruch 1, **dadurch gekennzeichnet, dass** die nachweisbaren Verbindungen kationischen Typs der folgenden Formel entsprechen: 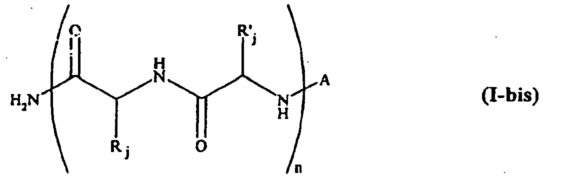 in welcher:
- n eine ganze, von 2 bis 10 variierende Zahl darstellt, und vor allem von 2 bis 6, und die vorzugsweise gleich 2 oder 3 ist,
- j der in Anspruch 1 gegebenen Definition entspricht,
- Rⱼ und R'ⱼ eine seitliche Aminosäurenkette darstellen, die **dadurch gekennzeichnet sind, dass** mindestens zwei der Rⱼ-Gruppen eine seitliche Aminosäurenkette mit basischem Charakter darstellen und **dadurch**, dass alle R'ⱼ von einer seitlichen Aminosäurenkette mit basischem Charakter unterschiedlich sind, und
- A wie in Anspruch 1 definiert ist.

5. Verwendung nach einem der Ansprüche 1 bis 4, **dadurch gekennzeichnet**, das die nachweisbaren Verbindungen kationischen Typs in Form von Salzen verwendet werden.

6. Verwendung nach einem der Ansprüche 1 bis 5 für die *in vitro*-Determinierung von Liganden eines Rezeptors, von denen kein Ligand bekannt ist oder von denen kein Ligand bekannt ist, der für die Verbindungstest von spezifischer Affinität verwendbar ist.

7. Verfahren zur Auswahl von Liganden von Rezeptoren, von denen kein Ligand bekannt ist oder von denen kein verwendbarer Ligand bekannt ist, wobei das Verfahren die folgenden Schritte umfasst:
- die Verwendung nach einem der Ansprüche 1 bis 5, die das Zusammenbringen einer Kollektion nachvollziehbarer Verbindungen mit Zellen impliziert, die durch eine Konstruktion transfiziert wurden, die die Fusion der für ein fluoreszierendes Protein kodierenden Sequenz mit der für einen Rezeptor kodierenden Nukleotidsequenz enthält, von dem kein verwendbarer Ligand bekannt ist, und das Mischen der Zellen und der Kollektion,
- den Nachweis der Fluoreszenz des Gemischs durch Erregung des fluoreszierenden Proteins und Messen der Sendefluoreszenz des fluoreszierenden Proteins und die Bestimmung des Löschprozentsatzes der Fluoreszenz durch Vergleich der Sendefluoreszenz des fluoreszierenden Proteins in dem Gemisch mit der durchschnittlichen Fluoreszenz des fluoreszierenden Proteins bei Liganden-Abwesenheit,
wobei die durchschnittliche Fluoreszenz des fluoreszierenden Proteins bei Liganden-Abwesenheit durch Kontrolltests gemessen wird, die der Messung der Fluoreszenz des fluoreszierenden Proteins bei Abwesenheit der Kollektion von Verbindungen entspricht, und
- die Bestimmung der Verbindungen, die einen Löschprozentsatz der Fluoreszenz des fluoreszierenden Proteins von mindestens 5 % und ihre Identifikation als Ligand ergeben.

8. Verfahren nach Anspruch 7, **dadurch gekennzeichnet, dass** der Rezeptor aus den mit G-Proteinen (RCPG) gekoppelten Rezeptoren ausgewählt ist.

9. Verfahren nach Anspruch 7, **dadurch gekennzeichnet, dass** der Rezeptor aus GPR50, GPR37, GPR19, GPR15, GPR31, GPR81, GPR3 und EDG7, dem Rezeptor APJ, FPRL1, den Chimokin-Rezeptoren CXCR1, CXCR2, CXCR3, CXCR4, den Glucagon-like Peptid-Rezeptoren (GLP-1R und GLP-2R), den Rezeptoren CRF1 und CRF2, den glutamatmetabotropen Rezeptoren (mGluR) und den GABA-Rezeptoren ausgewählt ist.

10. Verfahren nach einem der Ansprüche 8 oder 9, **dadurch gekennzeichnet, dass** der Rezeptor aus den an orphane G-Proteine gekoppelten Rezeptoren ausgewählt ist.
